(19) Europäisches Patentamt
European Patent Office
Office européen des brevets

(11) EP 4 644 875 A1

# (12) EUROPEAN PATENT APPLICATION
published in accordance with Art. 153(4) EPC

(43) Date of publication:
**05.11.2025 Bulletin 2025/45**

(21) Application number: **24759964.0**

(22) Date of filing: **16.01.2024**

(51) International Patent Classification (IPC):
***G01N 21/78** (2006.01)* ***G01N 33/49** (2006.01)*
***G01N 33/52** (2006.01)*

(52) Cooperative Patent Classification (CPC):
**G01N 21/78; G01N 21/3151; G01N 21/8483; G01N 33/49; G01N 33/52;** G01N 2021/3144; G01N 2021/3148

(86) International application number:
**PCT/JP2024/000993**

(87) International publication number:
**WO 2024/176657 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.02.2023 JP 2023026711**

(71) Applicant: **Terumo Kabushiki Kaisha**
**Tokyo 151-0072 (JP)**

(72) Inventors:
- **AIKAWA Ryokei**
  **Ashigarakami-gun, Kanagawa 259-0151 (JP)**
- **IKEDA Tomohiro**
  **Ashigarakami-gun, Kanagawa 259-0151 (JP)**
- **TAKAGI Shun**
  **Ashigarakami-gun, Kanagawa 259-0151 (JP)**

(74) Representative: **TBK**
**Bavariaring 4-6**
**80336 München (DE)**

## (54) COMPONENT MEASUREMENT DEVICE AND COMPONENT MEASUREMENT METHOD

(57) A component measurement device according to the present disclosure is a component measurement device for measuring a measurement target component in blood based on optical characteristics of a coloring component generated by a reaction between the measurement target component in the blood and a reagent, and the component measurement device is configured to correct a measured value of the measurement target component based on an oxygen saturation of the blood.

*FIG. 1*

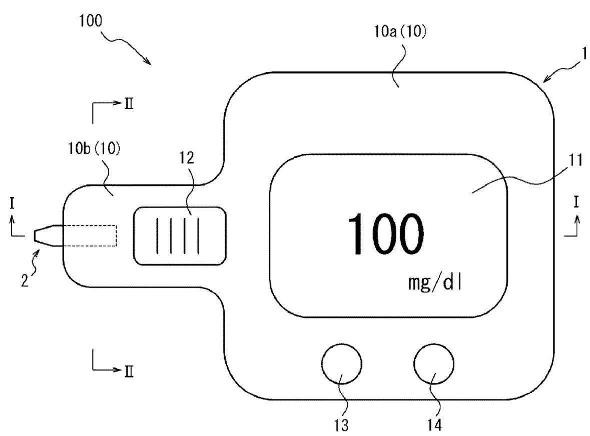


EP 4 644 875 A1

## Description

### Technical Field

The present disclosure relates to a component measurement device and a component measurement method.

### Background Art

Conventionally, in the biochemical field or the medical field, as a method for measuring a measurement target component contained in blood (whole blood) as a specimen, a method is known in which blood is separated into a portion containing the measurement target component and a portion not containing the measurement target component, and the amount or concentration of the measurement target component is measured. For example, in order to measure the glucose concentration (mg/dL, mmol/L) in the plasma, there is a method of measuring the glucose concentration in the plasma by a step of separating a plasma component from the blood using a filter or the like.

It is, however, difficult to completely separate the plasma component in the blood in a short time, and further, performance of a filter or the like used for separation varies. Therefore, a hemocyte component may be partially contained in the separated plasma component, and it is difficult to accurately measure the glucose concentration.

Meanwhile, as a method for measuring a measurement target component in blood without separating the measurement target component from the blood, whole blood measurement using an absorptiometry is known. This method can shorten the time required for measuring the measurement target component as compared with the above-described method of performing the step of separating the measurement target component. However, when the blood contains a large amount of a component other than the measurement target component, the component may cause an optical phenomenon such as light absorption and light scattering, and as a result, may act as a disturbance factor (noise) in the measurement. Therefore, in order to maintain the measurement accuracy for the measurement target component, it is necessary to eliminate the influence of the disturbance factor, and various methods for eliminating the influence of the disturbance factor have been proposed.

For example, Patent Literature 1 discloses a component measurement device and a component measurement method for measuring a glucose concentration in a plasma component, in which an amount of influence of a disturbance factor at a measurement wavelength is estimated from a measured value in a long wavelength region on a longer wavelength side than the measurement wavelength using a plurality of types of light sources, a measured value at the measurement wavelength is corrected using the estimated amount of influence of the disturbance factor, and then the measured value at the measurement wavelength is further corrected using a predicted hematocrit value.

### Citation List

#### Patent Literature

Patent Literature 1: WO 2018/173609 A

### Summary of Invention

#### Technical Problem

In the whole blood measurement using the absorptiometry, however, it is required to eliminate a larger amount of influence of the disturbance factor and to further improve the accuracy of component measurement.

An object of the present disclosure made in view of such circumstances is to provide a component measurement device and a component measurement method that improve the measurement accuracy of whole blood measurement that is performed using an absorptiometry.

#### Solution to Problem

[1] A component measurement device according to an embodiment of the present disclosure is a component measurement device for measuring a measurement target component in blood using a reagent that reacts with the measurement target component in the blood based on optical characteristics of a coloring component generated by a coloring reaction between the measurement target component and the reagent, and the component measurement device is configured to correct a measured value of the measurement target component based on an oxygen

saturation of the blood.

[2] A component measurement device according to an embodiment of the present disclosure is the component measurement device according to [1], the component measurement device preferably includes two correction light sources including a first correction light source that emits an irradiation light beam having a wavelength at which an absorption coefficient of deoxyhemoglobin is equal to an absorption coefficient of oxyhemoglobin, and a second correction light source that emits an irradiation light beam having a wavelength at which an absorption coefficient of deoxyhemoglobin is different from an absorption coefficient of oxyhemoglobin, and a light receiving unit that receives, of the irradiation light beams from the two correction light sources, a light beam that has passed through the blood as a transmission light beam, and the component measurement device is preferably configured to estimate the oxygen saturation of the blood based on the transmission light beam emitted from the two correction light sources and received by the light receiving unit.

[3] A component measurement device according to an embodiment of the present disclosure is the component measurement device according to [2], and the component measurement device is preferably configured to determine whether or not a reaction between the measurement target component and the reagent is started based on a time-series change in the transmission light beam emitted from at least one of the two correction light sources and received by the light receiving unit, and is preferably configured to estimate the oxygen saturation of the blood based on the transmission light beam emitted from the two correction light sources and received by the light receiving unit before start of the reaction.

[4] A component measurement device according to an embodiment of the present disclosure is the component measurement device according to [2] or [3], the component measurement device preferably includes a first light source that emits an irradiation light beam having a measurement wavelength within a wavelength range corresponding to a full width at half maximum of a peak wavelength region in an absorbance spectrum of the coloring component, a second light source that emits an irradiation light beam having a second wavelength and a third light source that emits an irradiation light beam having a third wavelength, the second wavelength and the third wavelength belonging to a longer wavelength region than the measurement wavelength, and a fourth light source that emits an irradiation light beam having a fourth wavelength and a fifth light source that emits an irradiation light beam having a fifth wavelength, the fourth wavelength and the fifth wavelength belonging to a shorter wavelength region than the measurement wavelength that belongs to the wavelength range corresponding to the full width at half maximum, the component measurement device is preferably configured to receive, by the light receiving unit, among the irradiation light beams from the first light source to the fifth light source, a light beam that has passed through the blood as a transmission light beam, and is preferably configured to measure the measurement target component in the blood using a measured value of absorbance of a mixture containing the blood, the reagent, and the coloring component at the measurement wavelength and measured values of absorbances of the mixture at the second wavelength to the fifth wavelength, the first correction light source is preferably the fourth light source, and the second correction light source is preferably the first light source.

[5] A component measurement device according to an embodiment of the present disclosure is the component measurement device according to any one of [1] to [4], in which the reagent preferably contains, in addition to an oxidoreductase that specifically reacts with the measurement target component and a color-developing reagent that causes coloring according to an amount of reaction between the measurement target component and the oxidoreductase, a hemolysis reagent that hemolyzes the blood and an oxidation reagent that oxidizes hemoglobin ($Fe^{2+}$) in the blood.

[6] A component measurement method according to an embodiment of the present disclosure is a component measurement method for measuring a measurement target component in blood in a mixture of the measurement target component in the blood and a reagent based on optical characteristics of a coloring component generated by a coloring reaction between the measurement target component and the reagent, and the component measurement method includes correcting a measured value of the measurement target component based on an oxygen saturation of the blood.

Advantageous Effects of Invention

According to the present disclosure, it is possible to provide a component measurement device and a component measurement method that improve the measurement accuracy of whole blood measurement that is performed using an absorptiometry.

Brief Description of Drawings

Fig. 1 is a top view of a component measurement device set in which a component measurement chip is attached to a component measurement device according to an embodiment of the present disclosure.

Fig. 2 is a diagram illustrating a cross section taken along line I-I in Fig. 1.

Fig. 3 is a diagram illustrating a cross section taken along line II-II in Fig. 1.

Fig. 4 is a top view illustrating the component measurement chip illustrated in Fig. 1.

Fig. 5 is a cross-sectional view taken along line III-III in Fig. 4.

Fig. 6 is an electrical block diagram of the component measurement device illustrated in Fig. 1.

Fig. 7 is a functional block diagram of an arithmetic unit illustrated in Fig. 6.

Fig. 8 is a diagram illustrating a positional relationship among a plurality of light sources in the component measurement device illustrated in Fig. 1.

Fig. 9 is a diagram illustrating irradiation positions from the plurality of light sources illustrated in Fig. 8 on a mixture.

Fig. 10 is a diagram illustrating absorbance spectra of six types of mixtures obtained by subjecting each of six types of blood specimens to a coloring reaction with a measurement reagent.

Fig. 11 is a diagram illustrating absorbance spectra of seven types of blood specimens.

Fig. 12 is a diagram illustrating an absorption coefficient of deoxyhemoglobin and an absorption coefficient of oxyhemoglobin.

Fig. 13 is a diagram illustrating a ratio of the absorption coefficient of oxyhemoglobin to the absorption coefficient of deoxyhemoglobin.

Fig. 14 is a graph illustrating an occupancy of a long wavelength region and an occupancy of a short wavelength region in an absorbance that is caused by a disturbance factor (noise) other than a coloring component at a measurement wavelength and is estimated by regression analysis.

Fig. 15 is a schematic view schematically illustrating stages in which blood is developed on a component measurement chip.

Fig. 16 is a schematic view schematically illustrating a change in absorbance accompanying development of blood on the component measurement chip.

Fig. 17 is a flowchart illustrating a method of correction for a measurement target component according to an embodiment of the present disclosure.

Fig. 18 is a flowchart illustrating a component measurement method according to an embodiment of the present disclosure.

Description of Embodiments

In the following, a component measurement device 1 according to an embodiment of the present disclosure will be described with reference to the drawings. In the drawings, the same or corresponding portions are denoted by the same reference signs. However, it should be noted that the drawings are schematic, and ratios of dimensions and the like may be different from actual ones.

First, the component measurement device 1 according to the present embodiment will be described. Fig. 1 is a top view illustrating a component measurement device set 100 in which a component measurement chip 2 is attached to the component measurement device 1 according to the present embodiment. Fig. 2 is a cross-sectional view illustrating a cross section taken along line I-I in Fig. 1. Fig. 3 is a cross-sectional view illustrating a cross section taken along line II-II in Fig. 1. Figs. 2 and 3 are enlarged views of the vicinity of a portion to which the component measurement chip 2 is attached.

As illustrated in Figs. 1 to 3, the component measurement device set 100 includes the component measurement device 1 and the component measurement chip 2. The component measurement device 1 of the present embodiment is a blood glucose level measurement device capable of measuring the concentration of glucose in a plasma component as a measurement target component in blood. The component measurement chip 2 of the present embodiment is a blood glucose level measurement chip that can be attached to an end of the blood glucose level measurement device as the component measurement device 1. The term "blood" as used herein means whole blood that is not separated into individual components and contains all the components.

The component measurement device 1 includes a housing 10 made of a resin material, buttons provided on an upper surface of the housing 10, a display unit 11 provided on the upper surface of the housing 10 and composed of liquid crystal, a light emitting diode (LED), or the like, and a detachment lever 12 operated when the component measurement chip 2 attached to the component measurement device 1 is detached. The buttons of the present embodiment may include a power button 13 and an operation button 14.

As illustrated in Fig. 1, the housing 10 includes a main body portion 10a having an upper surface on which the buttons and the display unit 11 described above are disposed and having a substantially rectangular outer shape in top view, and a chip attachment portion 10b protruding outward from the main body portion 10a and having an upper surface on which the detachment lever 12 is disposed. As illustrated in Fig. 2, a chip attachment space S having a distal end opening formed on a distal end surface of the chip attachment portion 10b as one end is defined in the chip attachment portion 10b.

The component measurement chip 2 is inserted into the chip attachment space S from the outside of the chip attachment portion 10b through the distal end opening, and is pushed to a predetermined position, thereby being locked to the chip attachment portion 10b of the component measurement device 1. In this way, the component measurement chip 2 is attached to the component measurement device 1. The component measurement chip 2 can be locked to the component measurement device 1 by various configurations, for example, by forming a claw portion that can be engaged with a part of the component measurement chip 2 in the chip attachment portion 10b.

Meanwhile, the locked state of the component measurement chip 2 by the chip attachment portion 10b of the component measurement device 1 is released by operating the detachment lever 12 described above from the outside of the housing 10 in a state where the component measurement chip 2 is attached to the component measurement device 1. Then, an eject pin 26 (see Fig. 2) in the housing 10 moves in conjunction with the release of the locked state of the component measurement chip 2, and the component measurement chip 2 becomes detachable from the component measurement device 1.

Note that, as illustrated in Fig. 1, the housing 10 of the present embodiment has a configuration including the main body portion 10a having a substantially rectangular shape in top view and the chip attachment portion 10b protruding outward from the main body portion 10a, but the shape of the housing 10 is not limited to that of the present embodiment as long as the housing 10 includes a chip attachment portion to which the component measurement chip 2 can be attached. Therefore, in the component measurement device 1, in addition to the shape of the housing 10 of the present embodiment, any shape that makes it easy for a user to grip the housing 10 with one hand can be adopted.

The display unit 11 is configured to display information on the measurement target component measured by the component measurement device 1. In the present embodiment, a glucose concentration measured by the component measurement device 1 as a blood glucose level measurement device can be displayed on the display unit 11. The display unit 11 may be configured to be capable of displaying, in addition to the information on the measurement target component, various types of information such as measurement conditions of the component measurement device 1 or instruction information that instructs a user to perform a predetermined operation. The user can operate the power button 13 or the operation button 14 while checking contents displayed on the display unit 11.

As illustrated in Figs. 2 and 3, the component measurement device 1 includes a light emitting unit 66 and a light receiving unit 72. The light emitting unit 66 and the light receiving unit 72 are disposed so as to face each other with the chip attachment space S interposed therebetween. As illustrated in Figs. 2 and 3, in a state where the component measurement chip 2 is attached to the chip attachment space S of the component measurement device 1, the component measurement chip 2 is irradiated with an irradiation light beam emitted by the light emitting unit 66. The light receiving unit 72 receives a transmission light beam that has passed through the component measurement chip 2 among irradiation light beams emitted from the light emitting unit 66 to the component measurement chip 2. The light receiving unit 72 can also measure the amount of irradiation light emitted from the light emitting unit 66 in a state where the component measurement chip 2 is not attached, and correct an amount of change of the light from the light emitting unit with the amount of irradiation light used as an initial value.

As illustrated in Figs. 2, 3, and 6, the light emitting unit 66 includes five light sources. Specifically, the light emitting unit 66 includes a first light source 67, a second light source 68a, a third light source 68b, a fourth light source 68c, and a fifth light source 68d. Here, as illustrated in Fig. 3, the first light source 67, the second light source 68a, and the third light source 68b are disposed at different positions in a flow path width direction B (a horizontal direction in Fig. 3) orthogonal to a flow direction A (a direction toward the right in Fig. 2) in which blood flows in a flow path 23 of the component measurement chip 2 described later. Details of disposition of the first light source 67 to the fifth light source 68d will be described later (see Fig. 8).

Next, the component measurement chip 2 according to the present embodiment will be described. Fig. 4 is a top view illustrating the component measurement chip 2. Fig. 5 is a cross-sectional view taken along line III-III in Fig. 4. As illustrated in Figs. 4 and 5, the component measurement chip 2 includes a base member 21 having a substantially rectangular plate-like outer shape, a measurement reagent 22 held by the base member 21, and a cover member 25 covering the base member 21.

A groove is formed in an outer surface on one side in a thickness direction of the base member 21 (the direction is the same as a thickness direction C of the component measurement chip 2 illustrated in Figs. 2 and 3 in the present embodiment, and therefore will be hereinafter referred to as the thickness direction C). The groove of the base member 21 forms a hollow portion extending in a direction orthogonal to the thickness direction C by being covered with the cover member 25. The hollow portion constitutes the flow path 23 of the component measurement chip 2. A supply portion 24 capable of supplying blood from the outside is formed at one end of the flow path 23. The measurement reagent 22 is held in a groove bottom of the groove of the base member 21 on an inner wall of the flow path 23. The measurement reagent 22 of the present embodiment is applied to the groove bottom of the groove as the flow path 23, but the present invention is not limited thereto. Blood supplied from the outside to the supply portion 24 moves in the flow direction A along the flow path 23 using, for example, a capillary phenomenon, reaches a holding position where the measurement reagent 22 is held, and comes into contact with the measurement reagent 22. The measurement reagent 22 contains a color-developing reagent

that develops color by a coloring reaction with blood. Therefore, when the measurement reagent 22 and blood come into contact with each other, a coloring reaction in which the color-developing reagent contained in the measurement reagent 22 develops color occurs to generate a mixture X containing a coloring component (see, for example, Fig. 2). As will be described in detail later, the measurement reagent 22 may contain a reagent other than the color-developing reagent.

In addition, a gap 23a is formed between the cover member 25 and the measurement reagent 22. The blood that moves in the flow direction A through the flow path 23 from the supply portion 24 reaches the other end of the flow path 23 through the gap 23a. Therefore, the blood can come into contact with the measurement reagent 22 in the entire region of the flow direction A to cause the coloring reaction.

In Fig. 2, for convenience of description, the holding position of the measurement reagent 22 is illustrated as the "mixture X", but the mixture X is located not only at the holding position of the measurement reagent 22 but also in the vicinity of the holding position of the measurement reagent 22, such as the gap 23a. More specifically, the blood that enters the flow path 23 from the supply portion 24 reaches a downstream end of the flow path 23 through the gap 23a while being in contact with the measurement reagent 22 at the holding position, and the flow path 23 is filled with the blood. Then, a coloring reaction of the measurement reagent 22 with the blood proceeds, and the mixture X is located at the holding position and in the vicinity thereof.

The flow path 23 of the present embodiment is formed of a hollow portion defined by the base member 21 and the cover member 25, but the configuration of the flow path is not limited thereto. The flow path may be formed only of a groove formed on an outer surface on one side of the base member 21 in the thickness direction.

The base member 21 and the cover member 25 are preferably made of a transparent material in order to obtain a signal sufficient for measurement as the amount of transmission light after an irradiation light beam passes through the base member 21 and the cover member 25. Examples of the materials of the base member 21 and the cover member 25 include: a transparent organic resin material such as polyethylene terephthalate (PET), polymethyl methacrylate (PMMA), polystyrene (PS), cyclic polyolefin (COP), a cyclic olefin copolymer (COC), or polycarbonate (PC); and a transparent inorganic material such as glass or quartz.

The measurement reagent 22 contains an enzyme that reacts with the measurement target component in blood and a color-developing reagent that causes coloring according to the concentration of the measurement target component in blood. The measurement reagent 22 of the present embodiment contains an enzyme that specifically reacts with glucose as a measurement target component in blood, and a color-developing reagent that causes quantitative coloring by electrons generated by the reaction between the enzyme and glucose. Examples of the measurement reagent 22 of the present embodiment include: a mixed reagent of (i) glucose oxidase (GOD), (ii) peroxidase (POD), (iii) 1-(4-sulfophenyl)-2,3-dimethyl-4-amino-5-pyrazolone, and (iv) N-ethyl-N-(2-hydroxy-3-sulfopropyl)-3,5-dimethylaniline sodium salt monohydrate (MAOS); and a mixed reagent of glucose dehydrogenase (GDH) and a tetrazolium salt. The measurement reagent 22 may contain an electron mediator or the like as necessary.

Examples of the enzyme of the present embodiment include an oxidoreductase. Here, the oxidoreductase is not particularly limited, and can be appropriately selected according to the type of biological component to be measured. Specific examples thereof include glucose dehydrogenase (GDH) such as glucose dehydrogenase (GDH), glucose dehydrogenase (PQQ-GDH) using pyrroloquinoline quinone (PQQ) as a coenzyme, glucose dehydrogenase (FAD-GDH) using flavin adenine dinucleotide (FAD) as a coenzyme, glucose dehydrogenase (NAD-GDH) using nicotinamide adenine dinucleotide (NAD) as a coenzyme, or glucose dehydrogenase (NADP-GDH) using nicotinamide adenine dinucleotide phosphate (NADP) as a coenzyme, glucose oxidase (GOD), lactic acid dehydrogenase (LDH), cholesterol dehydrogenase, cholesterol oxidase, and uric acid dehydrogenase. Here, the oxidoreductase may be used singly or in combination of two or more types thereof. For example, when the measurement target is glucose, the oxidoreductase is preferably glucose dehydrogenase or glucose oxidase. In addition, when cholesterol is measured, the oxidoreductase is preferably cholesterol dehydrogenase or cholesterol oxidase. When the measurement reagent 22 contains an oxidoreductase, the content of the oxidoreductase in the measurement reagent 22 is not particularly limited, and can be appropriately selected according to the amount of the color-developing reagent. The enzyme of the present embodiment is preferably glucose dehydrogenase.

As the color-developing reagent of the present embodiment, a pigment compound in which a peak wavelength in an absorbance spectrum of a coloring component generated by a coloring reaction with glucose in the blood is different from a peak wavelength caused by light absorption characteristics of hemoglobin in a hemocyte is used. In the color-developing reagent of the present embodiment, the absorbance spectrum of the coloring component generated by the coloring reaction with glucose in the blood has a peak wavelength around 650 nm, but the present invention is not limited thereto. Details will be described later.

The color-developing pigment of the present embodiment is preferably a tetrazolium salt, and more preferably 2-benzothiazolyl-3-(4-carboxy-2-methoxyphenyl)-5-[4-(2-sulfoethylcarbamoyl)phenyl]-2H-tetrazolium or a tetrazolium salt described in WO 2018/051822 A. According to a more preferable embodiment of the present disclosure, the color-developing pigment is at least one selected from the group consisting of 2-benzothiazolyl-3-(4-carboxy-2-methoxyphenyl)-5-[4-(2-sulfoethylcarbamoyl)phenyl]-2H-tetrazolium and the tetrazolium salt represented by the following formula (1).

According to a particularly preferable embodiment of the present disclosure, the color-developing pigment is the tetrazolium salt represented by the following formula (1).

[Chem. 1]

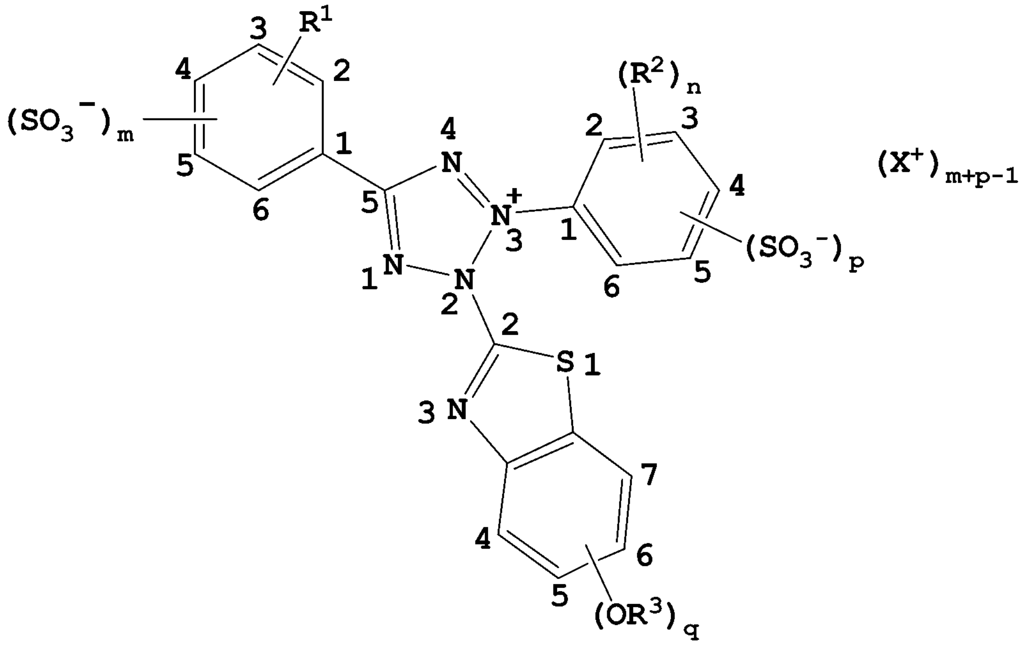

(1)

In the above formula (1), $R^1$ represents any one selected from the group consisting of a hydrogen atom, a hydroxy group, a methoxy group, and an ethoxy group, $R^2$ represents any one selected from the group consisting of a nitro group, $-OR^4$, and a carboxyl group, $R^3$ represents a hydrogen atom, a methyl group, or an ethyl group, in which at least one $R^3$ is a methyl group or an ethyl group, $R^4$ represents a methyl group or an ethyl group, m represents the number of sulfo groups ($-SO_3^-$) bonded to a phenyl group at a 5-position of a tetrazole skeleton, and is 1 or 2, n represents the number of $R^2$s bonded to a phenyl group at a 3-position of the tetrazole skeleton, and is an integer of 0 to 2, p represents the number of sulfo groups ($-SO_3^-$) bonded to a phenyl group at a 3-position of the tetrazole skeleton, and is 0 or 1, n + p is equal to or more than 1, q is 1 or 2, when q is 2, $OR^3$s are disposed adjacent to each other, in which $OR^3$s may form a ring with each other, and X represents a hydrogen atom or an alkali metal.

In addition to the enzyme that specifically reacts with the measurement target component, and the color-developing reagent, the measurement reagent 22 may contain a hemolysis reagent that hemolyzes the blood and an oxidation reagent that oxidizes hemoglobin ($Fe^{2+}$) in the blood to methemoglobin ($Fe^{3+}$).

Examples of the hemolysis reagent in the present embodiment include a nonionic surfactant, an amphoteric surfactant, and an anionic surfactant. A nonionic surfactant is preferable from a viewpoint of not inhibiting enzyme activity. The nonionic surfactant preferably has an HLB value of 11 or more and 15 or less (more preferably, 12 or more and 14 or less) from a viewpoint of a property of breaking the red blood cell membrane or solubility in water. Examples of such a nonionic surfactant include a polyoxyethylene alkyl ether in which the average number of moles of oxyethylene groups added is 1 or more and 150 or less and an alkyl group has 1 or more and 18 or less carbon atoms, and nonylphenyl polyethylene glycol. As such a nonionic surfactant, a synthesized product or a commercially available product may be used. Examples of the commercially available product include: a polyoxyethylene p-t-octylphenyl ether (Triton-based surfactant) such as polyoxyethylene (9) octylphenyl ether (octylphenoxy poly(ethyleneoxy)ethanol or octylphenyl-polyethylene glycol) (manufactured by Sigma-Aldrich Co., Ltd., Nonidet™ P-40), Triton (registered trademark) X-100 (polyoxyethylene (10) octylphenyl ether), or Triton (registered trademark) X-114 (polyoxyethylene (8) octylphenyl ether); a polyoxyethylene sorbitan fatty acid ester such as Tween (registered trademark) 85; Dodecyl-β-D-maltose; Octyl-β-D-glucoside; Nonidet (registered trademark) P-40 (octylphenoxy poly(ethyleneoxy)ethanol) and a substitute for Nonidet (registered trademark) P-40; Tergitol (registered trademark) NP-10 Surfactant (Nonylphenol Ethoxylate); IGEPAL (registered trademark) CA-630 (octylphenoxy poly(ethyleneoxy)ethanol); EMULGEN (registered trademark) 108 (polyoxyethylene lauryl ether) and EMULGEN (registered trademark) 109P; and Brij (registered trademark) 96 polyethylene glycol monooleyl ether (n = about 2). As the amphoteric surfactant, a synthesized product or a commercially available product may be used. Examples of the commercially available product include CHAPS (3-(3-cholamidepropyl) dimethylammonio-1-propanesulphonate), alkylpolyaminoethylglycine chloride; and sodium dodecyl sulfate. When the measurement reagent 22 contains a hemolysis reagent, the hemolysis reagent hemolyzes the blood to eliminate the influence of hemocytes, and the measurement accuracy for the measurement target component in the blood (in the present embodiment, the concentration measurement accuracy for glucose) can be improved.

The composition (content) of the hemolysis reagent in the measurement reagent 22 can be appropriately selected according to the sample amount (whole blood amount). The composition (content) (in terms of solid content) of the hemolytic agent in the measurement reagent 22 is, for example, 1 to 10% by volume with respect to 1.0 μL of a whole blood sample. In addition, the content of the hemolysis reagent in a shape of the component measurement chip 2 is 10 to 50% by mass, and preferably 20 to 40% by mass with respect to the total amount (solid content) of the measurement reagent. With such an amount, for example, a sensor that can sufficiently hemolyze a whole blood sample having a hematocrit of 20 to 70% can be obtained. In addition, when the measurement reagent 22 contains two or more types of hemolytic agents, the content of the hemolytic agent means the total amount of the hemolysis reagents to be blended.

Examples of the oxidation reagent of the present embodiment include nitrites. When the measurement reagent 22 contains an oxidation reagent, the oxidation reagent oxidizes deoxyhemoglobin (in particular, deoxyhemoglobin leaked from red blood cells due to hemolysis), so that it is possible to prevent false color development caused by the reaction of hemoglobin with the color-developing reagent. Examples of the nitrite include sodium nitrite, potassium nitrite, calcium nitrite, and ammonium nitrite. Sodium nitrite and potassium nitrite are preferable, and sodium nitrite is more preferable from a viewpoint of high stability and high versatility. The nitrite may be used singly or in combination of two or more types thereof.

The content of the nitrite in the measurement reagent 22 is, for example, 0.8 to 10.0 parts by mass, preferably 1.5 to 7.5 parts by mass, and more preferably 2.0 parts by mass or more and less than 5.0 parts by mass with respect to 100 parts by mass of the total amount (solid content) of the reagent. In addition, when the measurement reagent 22 contains two or more types of nitrites, the content of the nitrite means the total amount of the nitrites to be blended.

The content of the nitrite is preferably 50 mol or less with respect to 1 mol of the color-developing reagent.

The measurement reagent 22 may further contain a buffer such as a phosphate buffer, or various stabilizers. However, the measurement reagent 22 may be free of a hemolysis reagent, an oxidation reagent, or a buffer. The type and components of the reagent contained in the measurement reagent 22 are not limited thereto.

As illustrated in Fig. 2, when the component measurement device 1 measures the measurement target component, the component measurement chip 2 is attached into the chip attachment portion 10b. Then, when blood is supplied to the supply portion 24 formed at one end of the component measurement chip 2, the blood moves in the flow path 23 by, for example, a capillary phenomenon, and reaches a holding position where the measurement reagent 22 is held in the flow path 23, and glucose in the blood reacts with the measurement reagent 22 at the holding position. Then, at the holding position of the flow path 23, the mixture X containing the coloring component is generated. The component measurement device 1 of a so-called colorimetric type irradiates the mixture X containing the coloring component with an irradiation light beam, detects the amount of transmission light (or the amount of reflected light) of the irradiation light beam, and acquires a detection signal correlated with the intensity of color development according to the concentration in blood. Then, the component measurement device 1 refers to a calibration curve created in advance, and can thereby measure the measurement target component. As described above, the component measurement device 1 of the present embodiment measures the glucose concentration in a plasma component in blood.

Fig. 6 is an electrical block diagram of the component measurement device 1 illustrated in Figs. 1 to 3. Fig. 6 also illustrates a cross section of the component measurement chip 2 in a state of being attached to the component measurement device 1 (the same cross section as in Fig. 5) for convenience of description. In addition, in Fig. 6, an enlarged portion obtained by enlarging the vicinity of the component measurement chip 2 is separately illustrated in the upper left. Hereinafter, further details of the component measurement device 1 will be described.

As illustrated in Fig. 6, the component measurement device 1 further includes an arithmetic unit 60, a memory 62, a power circuit 63, and a measurement optical system 64 in addition to the above-described components.

The arithmetic unit 60 is constituted by a micro-processing unit (MPU) or a central processing unit (CPU), and can implement control operation of each unit by reading and executing a program stored in the memory 62 or the like. The memory 62 is constituted by a volatile or nonvolatile non-transitory storage medium, and is configured to be capable of reading or writing various types of data (including a program) necessary for executing the component measurement method described herein. The power circuit 63 supplies power to each unit in the component measurement device 1 including the arithmetic unit 60 or stops the supply thereof according to the operation of the power button 13.

The measurement optical system 64 is an optical system capable of acquiring optical characteristics of the mixture X containing the coloring component generated by a coloring reaction between glucose in blood and the color-developing reagent contained in the measurement reagent 22. Specifically, the measurement optical system 64 includes the light emitting unit 66, a light emission control circuit 70, the light receiving unit 72, and a light reception control circuit 74.

The light emitting unit 66 includes a plurality of light sources. Specifically, the light emitting unit 66 of the present embodiment includes five light sources that emit light beams (for example, visible light and infrared light) having different spectral radiation characteristics. More specifically, the light emitting unit 66 of the present embodiment includes the first

light source 67, the second light source 68a, the third light source 68b, the fourth light source 68c, and the fifth light source 68d. For convenience of description, Fig. 6 illustrates a positional relationship in which the five light sources including the first light source 67 to the fifth light source 68d are arranged in a line for illustrating the five light sources, but the positional relationship is different from the actual positional relationship among the first light source 67 to the fifth light source 68d. The actual positional relationship among the first light source 67 to the fifth light source 68d is the positional relationship illustrated in Figs. 2 and 3. The actual positional relationship among the first light source 67 to the fifth light source 68d will be described later in detail (see Fig. 8).

Peak wavelengths of light emitted from the first light source 67 to the fifth light source 68d are λ1 to λ5, respectively. As the first light source 67 to the fifth light source 68d, various light emitting elements such as a light emitting diode (LED) element, an organic electroluminescence (EL) element, an inorganic EL element, and a laser diode (LD) element can be applied. As the first light source 67 to the fifth light source 68d, the above-described LED element is easily used in consideration of versatility and the like. In the following, the above-described "peak wavelength" is described as the wavelength of light emitted from each light source, and for convenience of description, the peak wavelength λ1 of the first light source 67 is referred to as "first predetermined wavelength λ1", the peak wavelength λ2 of the second light source 68a is referred to as "second predetermined wavelength λ2", the peak wavelength λ3 of the third light source 68b is referred to as "third predetermined wavelength A3", the peak wavelength λ4 of the fourth light source 68c is referred to as "fourth predetermined wavelength A4", and the peak wavelength λ5 of the fifth light source 68d is referred to as "fifth predetermined wavelength A5".

As illustrated in Figs. 2 and 6, the light receiving unit 72 of the present embodiment is constituted by one light receiving element disposed so as to face the light emitting unit 66 with the component measurement chip 2 interposed therebetween. The light receiving unit 72 receives a transmission light beam that has been emitted from the first light source 67 to the fifth light source 68d of the light emitting unit 66 to the mixture X generated at the holding position of the measurement reagent 22 in the component measurement chip 2 and has passed through the component measurement chip 2. As the light receiving unit 72, various photoelectric conversion elements including a photo diode (PD) element, a photoconductor, and a photo transistor can be used.

The light emission control circuit 70 supplies a drive power signal to each of the first light source 67 to the fifth light source 68d to turn on or off the first light source 67 to the fifth light source 68d at predetermined intervals. The light reception control circuit 74 performs logarithmic conversion and A/D conversion on an analog signal output from the light receiving unit 72 to acquire a digital signal (hereinafter referred to as a detection signal).

Fig. 7 is a functional block diagram of the arithmetic unit 60 illustrated in Fig. 6. The arithmetic unit 60 implements functions of a measurement instruction unit 76 that gives instruction on a measurement operation by the measurement optical system 64 and a concentration measurement unit 77 that measures the concentration of the measurement target component using various data.

The concentration measurement unit 77 includes an absorbance acquisition unit 78, an absorbance correction unit 83, and a measurement target component calculation unit 84.

In Fig. 7, the memory 62 stores measured value data 85, correction coefficient data 86, and calibration curve data 90. The measured value data 85 includes a first measured value D1 to a fifth measured value D5 that are measured by the measurement optical system 64 and are absorbances of the mixture X at the first predetermined wavelength λ1 to the fifth predetermined wavelength λ5, respectively. The correction coefficient data 86 includes a group of correction coefficients correlated with absorbances of the mixture X at the second predetermined wavelength λ2 to the fifth predetermined wavelength A5, respectively. The calibration curve data 90 includes a calibration curve indicating a relationship between the absorbance of a coloring component in the mixture X obtained by correcting the absorbance of the mixture X measured at the first predetermined wavelength λ1 with the correction coefficient data 86 and various physical quantities (for example, glucose concentration), a calibration curve indicating a relationship between the absorbance of hemoglobin in the mixture X and a hematocrit value, or the like. The "hematocrit value" is a value indicating the volume ratio of a hemocyte component in blood to the blood (whole blood) in percentage.

As will be described in detail later, the component measurement device 1 measures the measurement target component in blood based on optical characteristics of the mixture X containing a coloring component generated by a coloring reaction between the measurement target component in blood and a reagent. Specifically, the component measurement device 1 estimates the amount of noise other than the coloring component included in the first measured value D1 of the absorbance of the mixture X measured by irradiating the mixture X with an irradiation light beam having the first predetermined wavelength λ1 as a measurement wavelength using irradiation light beams having the second predetermined wavelength λ2 to the fifth predetermined wavelength λ5, respectively. More specifically, the component measurement device 1 estimates the above-described noise amount using the second measured value D2 to the fifth measured value D5 of the absorbance of the mixture X measured by irradiating the mixture X with irradiation light beams having the second predetermined wavelength λ2 to the fifth predetermined wavelength λ5, respectively to measure the absorbance of the coloring component and further the measurement target component.

Here, Fig. 8 is a diagram illustrating a positional relationship among the first light source 67 that emits an

irradiation light beam having the first predetermined wavelength λ1 with which the mixture X is irradiated, the second light source 68a that emits an irradiation light beam having the second predetermined wavelength λ2 with which the mixture X is irradiated, the third light source 68b that emits an irradiation light beam having the third predetermined wavelength λ3 with which the mixture X is irradiated, the fourth light source 68c that emits an irradiation light beam having the fourth predetermined wavelength λ4 with which the mixture X is irradiated, and the fifth light source 68d that emits an irradiation light beam having the fifth predetermined wavelength λ5 with which the mixture X is irradiated. Fig. 8 illustrates a positional relationship among the first light source 67 to the fifth light source 68d when the component measurement device 1 is viewed from an upper surface (see Fig. 1) side thereof. In Fig. 8, for convenience of description, the position of the light receiving unit 72 in the flow path 23 of the component measurement chip 2 is indicated by a two-dot chain line, and in the present embodiment, the mixture X is generated at the holding position in the flow path 23 and in the vicinity thereof.

As illustrated in Figs. 2, 3, and 8, the first light source 67 to the fifth light source 68d are disposed so as to face the mixture X located in the flow path 23 of the blood. More specifically, the first light source 67 to the fifth light source 68d of the present embodiment are disposed so as to face the holding position of the measurement reagent 22 in the flow path 23 of the blood in a direction orthogonal to both the flow direction A and the flow path width direction B (the same direction as the thickness direction C of the component measurement chip 2 in the present embodiment).

As illustrated in Figs. 3 and 8, the first light source 67 and the second light source 68a are disposed side by side in the flow path width direction B orthogonal to the flow direction A of the blood at the position of the mixture X in the flow path 23 of the blood. With such a configuration, it is easy to set a first irradiation position SL1 (see Fig. 9) to be described later of the irradiation light beam from the first light source 67 on the mixture X and a second irradiation position SL2 (see Fig. 9) to be described later of the irradiation light beam from the second light source 68a on the mixture X at a position where the irradiation positions SL1 and SL2 at least partially overlap with each other in the flow path width direction B.

Here, Fig. 9 is a diagram illustrating the first irradiation position SL1 to a fifth irradiation position SL5 on the mixture X from the first light source 67 to the fifth light source 68d, respectively, when the component measurement device 1 is viewed from an upper surface (see Fig. 1) side thereof. As illustrated in Fig. 9, in the present embodiment, the first irradiation position SL1 of the irradiation light beam from the first light source 67 on the mixture X and the second irradiation position SL2 of the irradiation light beam from the second light source 68a on the mixture X overlap with each other in the flow path width direction B. With such a configuration, even if reaction unevenness occurs in the coloring reaction with the blood depending on the position in the flow direction A of the reagent due to the influence of the flow of the blood in the flow path 23, it is possible to reduce the variation in the measurement result due to the reaction unevenness. The above-described reaction unevenness is caused by the gradient of the hemocyte amount that can occur in the flow direction A. The gradient of the hemocyte amount in the flow direction A may occur due to dissolution of the measurement reagent 22 when the blood supplied from one end of the flow path 23 moves in the flow direction A and comes into contact with the measurement reagent 22 to cause a coloring reaction. When the measurement reagent 22 is dissolved, mainly the plasma component among the blood components is taken into the measurement reagent 22, and the mixture X is generated. As a result, the percentage of the hemocyte component increases around the mixture X. The blood travels in the flow direction A. Therefore, in the gap 23a, the hemocyte amount is larger on the downstream side than on the upstream side in the flow direction A. Specifically, a gradient of the hemocyte amount occurs in the gap 23a. The gradient of the hemocyte amount can cause the above-described reaction unevenness. The gradient of the hemocyte amount hardly occurs in the flow path width direction B.

As illustrated in Fig. 9, in the present embodiment, regions of the first irradiation position SL1 and the second irradiation position SL2 overlap with each other in the flow path width direction B. Specifically, in the present embodiment, the first irradiation position SL1 and the second irradiation position SL2 are at substantially equal positions in the flow direction A. However, the positional relationship between the first irradiation position SL1 and the second irradiation position SL2 is not limited to the above-described positional relationship, and it is only required that the first irradiation position SL1 and the second irradiation position SL2 have a positional relationship in which the irradiation positions SL1 and SL2 at least partially overlap with each other in the flow path width direction B. Meanwhile, when the regions of both the irradiation positions overlap with each other in the flow path width direction B as in the present embodiment, it is possible to further reduce the variation in the measurement result due to the reaction unevenness as compared with the configuration in which the regions overlap with each other only partially in the flow path width direction B.

Further, in the present embodiment, the first irradiation position SL1 and the second irradiation position SL2 not only overlap with each other in the flow path width direction B but also partially overlap with each other in the flow direction A. In this case, the first irradiation position SL1 and the second irradiation position SL2 can be made more coincident with each other, and it is possible to reduce the variation in the measurement result due to a difference in measurement positions in the mixture X. It is more preferable that regions of the first irradiation position SL1 and the second irradiation position SL2 overlap with each other in the flow direction A, that is, the first irradiation position SL1 and the second irradiation position SL2 are at substantially equal positions in the flow path width direction B.

In addition, in the present embodiment, as illustrated in Figs. 3 and 8, the first light source 67, the second light source 68a, and the third light source 68b are disposed side by side in the flow path width direction B with the first light

source 67 at the center. With such an arrangement, not only the two irradiation positions, that is, the first irradiation position SL1 from the first light source 67 and the second irradiation position SL2 from the second light source 68a, but also the two irradiation positions, that is, the first irradiation position SL1 and the third irradiation position SL3 from the third light source 68b can be easily set to positions where the irradiation positions at least partially overlap with each other in the flow path width direction B.

As illustrated in Fig. 9, in the present embodiment, the first irradiation position SL1 of the irradiation light beam from the first light source 67 on the mixture X and the third irradiation position SL3 of the irradiation light beam from the third light source 68b on the mixture X overlap with each other in the flow path width direction B. With such a configuration, similarly to the above-described relationship between the first irradiation position SL1 and the second irradiation position SL2, even if reaction unevenness occurs in the coloring reaction with the blood depending on the position in the flow direction A of the reagent due to the influence of the flow of the blood in the flow path 23, it is possible to reduce the variation in the measurement result due to the reaction unevenness.

As illustrated in Fig. 9, in the present embodiment, regions of the first irradiation position SL1 and the third irradiation position SL3 overlap with each other in the flow path width direction B. Specifically, in the present embodiment, the first irradiation position SL1 and the third irradiation position SL3 are at substantially equal positions in the flow direction A. However, the positional relationship between the first irradiation position SL1 and the third irradiation position SL3 is not limited to the above-described positional relationship, and it is only required that the first irradiation position SL1 and the third irradiation position SL3 have a positional relationship in which the irradiation positions SL1 and SL3 at least partially overlap with each other in the flow path width direction B. Meanwhile, when the regions of both the irradiation positions overlap with each other in the flow path width direction B as in the present embodiment, it is possible to further reduce the variation in the measurement result due to the reaction unevenness as compared with the configuration in which the regions overlap with each other only partially in the flow path width direction B.

In the present embodiment, the first irradiation position SL1 and the third irradiation position SL3 not only overlap with each other in the flow path width direction B but also partially overlap with each other in the flow direction A. In this case, the first irradiation position SL1 and the third irradiation position SL3 can be made more coincident with each other, and it is possible to reduce the variation in the measurement result due to a difference in measurement positions in the mixture X. It is more preferable that regions of the first irradiation position SL1 and the third irradiation position SL3 overlap with each other in the flow direction A, that is, the first irradiation position SL1 and the third irradiation position SL3 are at substantially equal positions in the flow path width direction B.

Here, as illustrated in Fig. 9, the second irradiation position SL2 and the third irradiation position SL3 of the present embodiment overlap with each other in the flow path width direction B. More specifically, regions of the second irradiation position SL2 and the third irradiation position SL3 of the present embodiment overlap with each other in the flow path width direction B. Alternatively, it is required that they at least partially overlap with each other in the flow path width direction B. Meanwhile, when the regions of both the irradiation positions overlap with each other in the flow path width direction B as in the present embodiment, it is possible to further reduce the variation in the measurement result due to the reaction unevenness as compared with the configuration in which the regions overlap with each other only partially in the flow path width direction B.

In the present embodiment, regions of the second irradiation position SL2 and the third irradiation position SL3 not only overlap with each other in the flow path width direction B but also partially overlap with each other in the flow direction A. In this case, the second irradiation position SL2 and the third irradiation position SL3 can be made more coincident with each other, and it is possible to reduce the variation in the measurement result due to a difference in measurement positions in the mixture X. It is more preferable that regions of the second irradiation position SL2 and the third irradiation position SL3 overlap with each other in the flow direction A, that is, the second irradiation position SL2 and the third irradiation position SL3 are at substantially equal positions in the flow path width direction B.

As described above, it is preferable that the first light source 67 to the third light source 68b are disposed side by side in the flow path width direction B, and regions of the first irradiation position SL1 to the third irradiation position SL3 overlap with each other in the flow path width direction B, and it is more preferable that regions of the first irradiation position SL1 to the third irradiation position SL3 overlap with each other also in the flow direction A.

In the present embodiment, the first light source 67 and the second light source 68a are disposed adjacent to each other in the flow path width direction B, and there is no gap in which another light source can be disposed between the first light source 67 and the second light source 68a. In addition, the first light source 67 and the third light source 68b are disposed adjacent to each other in the flow path width direction B, and there is no gap in which another light source can be disposed between the first light source 67 and the third light source 68b. As described above, the first light source 67, the second light source 68a, and the third light source 68b are disposed adjacent to each other without another light source interposed therebetween in the flow path width direction B. Therefore, it is easy to realize a configuration in which regions of the first irradiation position SL1, the second irradiation position SL2, and the third irradiation position SL3 overlap with each other in the flow direction A.

Next, a positional relationship among the first light source 67, the fourth light source 68c, and the fifth light source

68d will be described. As illustrated in Figs. 2 and 8, the first light source 67 and the fourth light source 68c of the present embodiment are disposed side by side in the flow direction A. In addition, as illustrated in Figs. 2 and 8, the first light source 67 and the fifth light source 68d of the present embodiment are disposed side by side in the flow direction A. More specifically, the first light source 67, the fourth light source 68c, and the fifth light source 68d are disposed side by side in the flow direction A with the first light source 67 at the center.

As described above, the second light source 68a and the third light source 68b are disposed adjacent to the first light source 67 in the flow path width direction B. In order to reduce the variation in the measurement result due to the flow of the blood in the flow path 23, the fourth light source 68c and the fifth light source 68d are also preferably disposed side by side with the first light source 67 in the flow path width direction B. However, when the fourth light source 68c and the fifth light source 68d are to be disposed side by side with the first light source 67 in the flow path width direction B, the first light source 67 and each of the fourth light source 68c and the fifth light source 68d cannot be disposed adjacent to each other due to the presence of the second light source 68a and the third light source 68b. Therefore, the distance between the first light source 67 and each of the fourth light source 68c and the fifth light source 68d in the flow path width direction B is larger than the distance between the first light source 67 and each of the second light source 68a and the third light source 68b in the flow path width direction B. When the distance is large, it is difficult to overlap regions of the first irradiation position SL1 from the first light source 67 and each of the fourth irradiation position SL4 from the fourth light source 68c and the fifth irradiation position SL5 from the fifth light source 68d with each other in the flow direction A. That is, the first irradiation position SL1 and each of the fourth irradiation position SL4 and the fifth irradiation position SL5 are likely to have a configuration in which the regions do not overlap with each other at all. In a case where regions of the first irradiation position SL1 and each of the fourth irradiation position SL4 and the fifth irradiation position SL5 do not overlap with each other, measurement points of the absorbance are different, and therefore the accuracy of the measurement result of the measurement target component may decrease. The regions of the first irradiation position SL1 from the first light source 67 and each of the fourth irradiation position SL4 from the fourth light source 68c and the fifth irradiation position SL5 from the fifth light source 68d can be overlapped with each other by inclining the fourth light source 68c and the fifth light source 68d. However, in such a case, a difference between an incident angle of the irradiation light beam from the first light source 67 on the mixture X and an incident angle of the irradiation light beam from each of the fourth light source 68c and the fifth light source 68d on the mixture X is large. When the difference in incident angle is large, the difference between an optical path length of the irradiation light beam from the first light source 67 in the mixture X and an optical path length of the irradiation light beam from each of the fourth light source 68c and the fifth light source 68d in the mixture X is large. Further, interface reflection of the irradiation light beam from the first light source 67 is different from interface reflection of the irradiation light beam from each of the fourth light source 68c and the fifth light source 68d. The difference in optical path length and the difference in interface reflection affect the measured value of absorbance. That is, the estimation accuracy of the noise amount in the measured value of absorbance from the irradiation light beam from the first light source 67 may decrease.

Therefore, in the present embodiment, the first light source 67 and the fourth light source 68c are disposed side by side in the flow direction A such that the region of the first irradiation position SL1 and the region of the fourth irradiation position SL4 overlap with each other in a state where a difference in incident angle on the mixture X is equal to or less than a predetermined value. More specifically, there is no gap in which another light source can be disposed between the first light source 67 and the fourth light source 68c in the flow direction A, and the first light source 67 and the fourth light source 68c are adjacent to each other in the flow direction A. Such a configuration is susceptible to the influence of the flow of the blood as compared with the configuration in which the light sources are disposed in the flow path width direction B. However, since the difference in incident angle is reduced and the irradiation positions are overlapped with each other, the estimation accuracy of the noise amount can be rather improved.

The first light source 67 and the fifth light source 68d are also disposed side by side in the flow direction A such that the region of the first irradiation position SL1 and the region of the fifth irradiation position SL5 can overlap with each other in a state where a difference in incident angle on the mixture X is equal to or less than a predetermined value. More specifically, there is no gap in which another light source can be disposed between the first light source 67 and the fifth light source 68d in the flow direction A, and the first light source 67 and the fifth light source 68d are adjacent to each other in the flow direction A.

As described above, the first light source 67 and each of the second light source 68a and the third light source 68b are adjacent to each other in the flow path width direction B. Therefore, the first irradiation position SL1 and each of the second irradiation position SL2 and the third irradiation position SL3 can be overlapped in a state where a difference in incident angle on the mixture X is equal to or less than a predetermined value. Specifically, the second light source 68a and the third light source 68b of the present embodiment are less susceptible to the influence of the flow of the blood owing to the relationship with the first light source 67, and regions of the irradiation positions can be overlapped with each other by reducing the difference in incident angle from the first light source 67.

Here, in the present embodiment, the second light source 68a and the third light source 68b that emit irradiation light beams having the second predetermined wavelength λ2 and the third predetermined wavelength A3, respectively, which have a large degree of influence on the estimation of the amount of noise included in the measured value of

absorbance measured using the irradiation light beam having the first predetermined wavelength λ1 from the first light source 67, are disposed side by side in the flow path width direction B with respect to the first light source 67. The fourth light source 68c and the fifth light source 68d that emit irradiation light beams having the fourth predetermined wavelength λ4 and the fifth predetermined wavelength λ5, respectively, which have a smaller degree of influence on the estimation of the noise amount than the second predetermined wavelength λ2 and the third predetermined wavelength A3 do, are disposed side by side in the flow direction A with respect to the first light source 67. With such an arrangement, the above-described estimation accuracy of the noise amount can be improved. Details of "degree of influence" on the estimation of the noise amount will be described later (see Fig. 14). As will be described in detail later, the above-described second predetermined wavelength λ2 and third predetermined wavelength A3 are wavelengths belonging to an infrared region, and the above-described fourth predetermined wavelength λ4 and fifth predetermined wavelength λ5 are wavelengths belonging to a visible region.

In addition, as illustrated in Figs. 2 and 3, the light receiving unit 72 faces the first light source 67 to the fifth light source 68d in the thickness direction C with the mixture X located in the flow path 23 of the attached component measurement chip 2 interposed therebetween, and receives, among the irradiation light beams from the first light source 67 to the fifth light source 68d, a light beam that has passed through the mixture X as a transmission light beam as described above. As illustrated in Figs. 2 and 3, the component measurement device 1 includes a first throttle portion 69a that is located between the mixture X and the light receiving unit 72 and adjusts the amount of light that reaches the light receiving unit 72 among transmission light beams that have passed through the mixture X. As described above, a difference between an incident angle of the irradiation light beam from the first light source 67 on the mixture X and an incident angle of the irradiation light beam from each of the second light source 68a to the fifth light source 68d on the mixture X affects estimation accuracy of the noise amount. Therefore, the difference between an incident angle of the irradiation light beam from the first light source 67 on the mixture X and an incident angle of the irradiation light beam from each of the second light source 68a to the fifth light source 68d on the mixture X is preferably small. That is, a distance T1 between the first light source 67 to the fifth light source 68d and the first throttle portion 69a in a facing direction (the same direction as the thickness direction C of the component measurement chip 2 in Figs. 2 and 3) is preferably long in order to improve the estimation accuracy of the noise amount. Meanwhile, by reducing a distance T2 between the first light source 67 to the fifth light source 68d and the light receiving unit 72 in a facing direction, light efficiency can be improved, and the component measurement device 1 can be downsized.

In addition, when a deviation between the region of the first irradiation position SL1 from the first light source 67 and the region of each of the second irradiation position SL2 to the fifth irradiation position SL5 of the second light source 68a to the fifth light source 68d (hereinafter, the deviation is referred to as "measurement visual field difference") is large, measurement points do not coincide with each other, and therefore the accuracy of the measurement result of the measurement target component may decrease. Therefore, the measurement visual field difference is preferably small. Therefore, a distance T3 between the mixture X and the first throttle portion 69a in a facing direction (the same direction as the thickness direction C of the component measurement chip 2 in Figs. 2 and 3) is preferably short.

Furthermore, as illustrated in Figs. 2 and 3, the component measurement device 1 includes a second throttle portion 69b that is located between the first light source 67 to the fifth light source 68d and the mixture X and adjusts the amount of light that reaches the mixture X from the first light source 67 to the fifth light source 68d. In particular, the second throttle portion 69b is preferably designed such that light reflected by an inner wall of the second throttle portion 69b (hereinafter, the light is referred to as "stray light") among light beams emitted from the first light source 67 to the fifth light source 68d does not enter the first throttle portion 69a. It can be considered that light emitted from the first light source 67 to the fifth light source 68d is attenuated to 5% by one time wall surface reflection and disappears by multiple reflection in which reflection is performed three or more times. Therefore, in the present embodiment, if the stray light reflected by the inner wall of the second throttle portion 69b does not reach the first throttle portion 69b and is reflected by a certain wall surface, the stray light does not enter the first throttle portion 69a by multiple reflection. In the present embodiment, an optical axis of each of the light sources is designed such that light is specularly reflected by the inner wall of the second throttle portion 69b, but actually, diffused reflection occurs on the inner wall of the second throttle portion 69b, and stray light also has a predetermined distribution. Therefore, in the present embodiment, it is preferable to set the above-described distance T4 and the like such that, even when a part of the stray light enters the first throttle portion 69a, a difference between an incident angle thereof and an incident angle from the first light source 67 is equal to or less than a predetermined value.

As described above, adjustment of the positions and the like of the first throttle portion 69a and the second throttle portion 69b can make the difference in incident angle between the irradiation light beams and the measurement visual field difference within a predetermined range. Incidentally, a lens such as a condenser lens is not used in the optical system of the component measurement device 1. When a lens is used, the lens can be brought close to the light source to improve the light collection efficiency. However, it is necessary to maintain the positional relationship between the light source and the lens with high accuracy, and high assembly accuracy is required, or an additional step of adjusting variation in the positional relationship between the light source and the lens is required. Therefore, in the component measurement device

1, a configuration is realized in which measurement accuracy is improved without requiring high assembly accuracy by setting the positions and the like of the first throttle portion 69a and the second throttle portion 69b without using a lens. However, a lens such as a condenser lens may be used in the optical system of the component measurement device 1.

As described above, in the component measurement device 1, by disposing the first light source 67 to the fifth light source 68d in a predetermined manner, the improvement of the estimation accuracy of the noise amount is realized while reducing the influence of the blood in the flow direction A in the flow path 23 of the blood.

To the component measurement device 1 of the present embodiment, the component measurement chip 2 can be attached. The component measurement chip 2 defines the flow path 23 through which blood flows and in which the measurement reagent 22 containing a color-developing reagent that undergoes a coloring reaction with the measurement target component in the blood is disposed. To the component measurement device 1 of the present embodiment, the component measurement chip 2 is attached, and the component measurement device 1 measures the measurement target component in the blood based on optical characteristics of the mixture containing a coloring component generated by a reaction with the measurement target component in the flow path 23. In addition, the component measurement device 1 includes the first light source 67, and the second light source 68a to the fifth light source 68d that emit irradiation light beams having the second predetermined wavelength $\lambda2$ to the fifth predetermined wavelength $\lambda5$, respectively. The first light source 67 emits an irradiation light beam having the first predetermined wavelength $\lambda1$, with which the mixture X in the flow path 23 of the component measurement chip 2 in a state of being attached to the component measurement device 1 is irradiated. The second light source 68a to the fifth light source 68d emit irradiation light beams having the second predetermined wavelength A2 to the fifth predetermined wavelength A5, respectively, with which the mixture X in the flow path 23 of the component measurement chip 2 in a state of being attached to the component measurement device 1 is irradiated. The irradiation light beams are used for estimation of the amount of noise caused by a disturbance factor other than a coloring component included in the measured value of absorbance of the mixture X measured using the amount of transmission light from the irradiation light beam from the first light source 67. Then, the first light source 67 to the third light source 68b are disposed side by side in the flow path width direction B orthogonal to the flow direction A of the blood at the position of the mixture X in the flow path 23 of the component measurement chip 2 in a state of being attached to the component measurement device 1.

As described above, the component measurement device 1 of the present embodiment measures the absorbance of the mixture X in the detachably attached component measurement chip 2, but the component measurement chip 2 does not have to require attachment and detachment. However, in consideration of user's convenience, environmental friendliness, and the like, it is preferable that a disposable component measurement chip 2 is detachably attached to a reusable component measurement device 1.

As illustrated in Fig. 8, the first light source 67 to the fifth light source 68d of the present embodiment are held by a thin plate-shaped holder member 80. The holder member 80 of the present embodiment has a cross-shaped outer shape in top view, and the first light source 67 is held at a central portion (cross portion) of the holder member 80 in top view. In addition, in the holder member 80, the second light source 68a is held at a position on one side in the flow path width direction B with respect to the central portion where the first light source 67 is held, and the third light source 68b is held at a position on the other side in the flow path width direction B. In addition, in the holder member 80, the fifth light source 68d is held at a position in the flow direction A with respect to the central portion where the first light source 67 is held, and the fourth light source 68c is held at an opposite position in the flow direction A.

Hereinafter, a component measurement method for measuring a measurement target component will be described. In the component measurement method, the coloring reaction between glucose as the measurement target component in blood and the color-developing reagent in the measurement reagent 22 is performed by using the blood (whole blood) and the color-developing reagent without separating the plasma component containing glucose from the blood, and the absorbance at a predetermined measurement wavelength of the coloring component generated by the coloring reaction between glucose and the color-developing reagent is estimated based on the absorbance at various wavelengths of the entire mixture X obtained by the coloring reaction.

First, referring to Figs. 10 and 11, problems in estimating a measurement target component in blood based on absorbance measurement using blood (whole blood) will be described. In the following examples, a reagent containing a tetrazolium salt (WST-4) as a color-developing reagent and mixed with glucose dehydrogenase (GDH) and an electron mediator was used as the measurement reagent 22.

Fig. 10 illustrates absorbance spectra of six types of mixtures X obtained by reacting each of six types of blood specimens each having a known hematocrit value and a known glucose concentration with the measurement reagent 22. These six types of blood specimens are referred to as first to sixth specimens. The first specimen has a hematocrit value of 20% and a glucose concentration of 0 mg/dL (denoted as "Ht20 bg0" in Fig. 10). The second specimen has a hematocrit value of 20% and a glucose concentration of 100 mg/dL (denoted as "Ht20 bg100" in Fig. 10). The third specimen has a hematocrit value of 20% and a glucose concentration of 400 mg/dL (denoted as "Ht20 bg400" in Fig. 10). The fourth specimen has a hematocrit value of 40% and a glucose concentration of 0 mg/dL (denoted as "Ht40 bg0" in Fig. 10). The fifth specimen has a hematocrit value of 40% and a glucose concentration of 100 mg/dL (denoted as "Ht40 bg100" in Fig.

10). The sixth specimen has a hematocrit value of 40% and a glucose concentration of 400 mg/dL (denoted as "Ht40 bg400" in Fig. 10).

In addition, Fig. 11 illustrates absorbance spectra of seven types of blood specimens each having a known hematocrit value and a known glucose concentration. These seven types of blood specimens are referred to as first to seventh specimens. The first to sixth specimens are the same as the first to sixth specimens illustrated in Fig. 10. The seventh specimen has a hematocrit value of 70% and a glucose concentration of 100 mg/dL. Since the absorbance spectra of blood specimens having the same hematocrit value substantially coincide with each other, Fig. 11 illustrates only three curves with different hematocrit values. Specifically, only three curves with hematocrit values of 20% (denoted as "Ht20" in Fig. 11), 40% (denoted as "Ht40" in Fig. 11), and 70% (denoted as "Ht70" in Fig. 11) are illustrated.

In general, when a component other than a coloring component to be measured for absorbance is contained in a sample, occurrence of an optical phenomenon may affect, as a disturbance factor (noise), the measurement result of the concentration of the measurement target component based on the absorbance of the coloring component. For example, "light scattering" due to a hemocyte component in blood, a surface of the component measurement chip, fine particles such as dust attached to the component measurement chip, and the like, or "light absorption" due to a dye component (specifically, hemoglobin) different from the coloring component to be measured occurs, and as a result, an absorbance larger than the true value tends to be obtained.

Specifically, the absorbance spectra of the blood specimens illustrated in Fig. 11 have two peaks centered around 540 nm and around 570 nm. These two peaks are mainly caused by light absorption of oxyhemoglobin in red blood cells. In addition, in the absorbance spectra of the blood specimens illustrated in Fig. 11, in the wavelength region of 600 nm or more, the absorbances gradually decrease substantially linearly as the wavelength becomes longer. The substantially linear portion is mainly caused by light scattering of the hemocyte component.

In other words, the absorbances of the blood specimens in the wavelength region on a longer wavelength side than around 600 nm are predominantly affected by light scattering due to a hemocyte component or the like. The absorbances of the blood specimens in the wavelength region on a shorter wavelength side than around 600 nm are affected more by light absorption due to hemoglobin than by light scattering due to a hemocyte component or the like.

The absorbance spectra of the mixture X illustrated in Fig. 10 have trend curves in which the absorbances gradually decrease as the wavelength becomes longer, similarly to the absorbance spectra of the blood illustrated in Fig. 11. However, in the absorbance spectra of the mixture X illustrated in Fig. 10, the absorbances increase around 600 nm to 700 nm, which is a wavelength region of visible light, as compared with the curves illustrated in Fig. 11. The absorbances increasing around 600 nm to 700 nm are mainly due to the light absorption characteristics of the coloring component generated by the coloring reaction between glucose in blood and the color-developing reagent in the measurement reagent 22.

In this way, in a case where an absorbance derived from the coloring component is accurately measured using the mixture X containing blood having the light absorption characteristics illustrated in Fig. 11 in addition to the coloring component to be measured, it is necessary to eliminate a disturbance factor (noise) such as light scattering due to a hemocyte component or the like or light absorption due to hemoglobin from the measured value of absorbance at a predetermined measurement wavelength (for example, 650 nm).

More specifically, it is necessary to estimate the amount of disturbance factor (noise) such as light scattering due to a hemocyte component or the like or light absorption due to hemoglobin at a predetermined measurement wavelength (for example, 650 nm) at which the light absorptivity of the coloring component to be measured is high, and to correct the measured value of absorbance at the predetermined measurement wavelength.

Hereinafter, details of the component measurement method executed by the component measurement device 1 will be described.

The component measurement device 1 measures the measurement target component in blood based on optical characteristics of the mixture X containing a coloring component generated by a coloring reaction between blood and the measurement reagent 22. Specifically, in the present embodiment, the component measurement device 1 measures the concentration of glucose contained in a plasma component in blood.

Then, the component measurement device 1 calculates the glucose concentration in the blood by correcting the measured value of absorbance of the mixture X at the measurement wavelength based on the optical characteristics due to the hemocyte component in the blood, the surface of the component measurement chip 2, or fine particles such as dust attached to the component measurement chip 2, and the ratio between deoxyhemoglobin and oxyhemoglobin in the red blood cells. In other words, the component measurement method by the component measurement device 1 includes a step of correcting the measured value of absorbance of the mixture X at the measurement wavelength based on information of scattered light due to the hemocyte component in the blood, the surface of the component measurement chip 2, or fine particles such as dust attached to the component measurement chip 2, and the ratio between deoxyhemoglobin and oxyhemoglobin in the red blood cells.

Further, in addition to the above-described correction, the component measurement device 1 is configured to perform correction of the glucose concentration in the blood based on the oxygen saturation of the blood. As a result, the

component measurement device 1 can correct the measured value of the measurement target component in consideration of the oxygen saturation different by the blood specimen, and consequently improve the measurement accuracy for the measurement target component in the blood.

Fig. 12 illustrates an absorption coefficient of deoxyhemoglobin (denoted as "Hb" in Fig. 12) and an absorption coefficient of oxyhemoglobin (denoted as "$HbO_2$" in Fig. 12). Hemoglobin in a red blood cell mainly contains oxyhemoglobin bonded to oxygen and deoxyhemoglobin in which oxygen is dissociated at a place where an oxygen partial pressure is small. Deoxyhemoglobin passes through the lung and is bonded to oxygen to generate oxyhemoglobin, oxyhemoglobin plays a role of transporting oxygen into the body through the artery, and a large amount of oxyhemoglobin can be observed in arterial blood. For example, when blood is collected from a pulp of a finger, the blood is capillary blood, and therefore contains a relatively large amount of oxyhemoglobin. Conversely, a large amount of deoxyhemoglobin can be observed in venous blood.

As an existing technique, it is common to correct the absorbance obtained at a measurement wavelength corresponding to a coloring component to be measured by using, for example, a hematocrit value without considering the ratio between deoxyhemoglobin and oxyhemoglobin. However, as illustrated in Fig. 12, the absorption coefficient of deoxyhemoglobin is not the same as the absorption coefficient of oxyhemoglobin, and the absorption amount by deoxyhemoglobin and the absorption amount by oxyhemoglobin vary depending on the wavelength. Fig. 13 illustrates a ratio of the absorption coefficient of oxyhemoglobin to the absorption coefficient of deoxyhemoglobin. For example, when the measurement wavelength for measuring the absorbance of the coloring component to be measured is 650 nm, the absorption coefficient of deoxyhemoglobin is about 0.9, and the absorption coefficient of oxyhemoglobin is about 0.09. That is, the absorption coefficient of oxyhemoglobin corresponds to about 10% of the absorption coefficient of total hemoglobin. In order to more accurately estimate the absorbance derived from the coloring component to be measured, it is important to consider the ratio between deoxyhemoglobin and oxyhemoglobin.

Therefore, in the component measurement device 1, the measurement wavelength for measuring the absorbance of the coloring component contained in the mixture X is defined as 650 nm, and the component measurement device 1 performs correction to eliminate, as a disturbance factor (noise), an influence of light scattering due to a hemocyte component or the like or an influence of light absorption due to hemoglobin in consideration of the ratio between deoxyhemoglobin and oxyhemoglobin from the measured value of absorbance of the mixture X measured at the measurement wavelength. Thus, the component measurement device 1 estimates the absorbance of the coloring component contained in the mixture X, and calculates the glucose concentration using a calibration curve indicating a relationship between the estimated absorbance and the glucose concentration.

Hereinafter, further details of the component measurement method executed by the component measurement device 1 will be described.

First, as for the color-developing reagent in the measurement reagent 22 used in the present embodiment, the absorbance of the coloring component generated by the coloring reaction with glucose in the blood has a peak around 600 nm, and in the present embodiment, the measurement wavelength at which the absorbance of the coloring component is measured is 605 nm.

As the measurement wavelength for measuring the absorbance of the coloring component to be measured, it is only required to use a wavelength at which the light absorptivity of the coloring component is relatively large and at which an influence of light absorption due to hemoglobin is relatively small. Specifically, it is only required to use a wavelength belonging to a wavelength range W3 (see Fig. 10 and Fig. 11) that corresponds to a full width at half maximum of a peak wavelength region in an absorbance spectrum of the coloring component to be measured and in which the ratio of an absorbance due to light absorption of hemoglobin to the total absorbance is relatively small. The wavelength range "corresponding to a full width at half maximum of a peak wavelength region" means a range from a wavelength indicating a half value on a short wavelength side to a wavelength indicating a half value on a long wavelength side when the full width at half maximum of the peak wavelength region in the absorbance spectrum is specified. In the absorbance spectrum of the coloring component to be measured in the present embodiment, the peak wavelength is around 600 nm, and the wavelength range corresponding to the full width at half maximum is about 500 nm to about 700 nm. In addition, the influence of light absorption due to hemoglobin in the total absorbance is relatively small in the wavelength region of 600 nm or more. Therefore, in the present embodiment, the wavelength range W3 that corresponds to a full width at half maximum of a peak wavelength region in an absorbance spectrum of the coloring component to be measured and in which the ratio of an absorbance due to light absorption of hemoglobin to the total absorbance is relatively small is 600 nm or more and 700 nm or less. Therefore, the measurement wavelength is not limited to 605 nm in the present embodiment, and another wavelength belonging to the range of 600 nm to 700 nm may be used as the measurement wavelength. The absorbance derived from the coloring component can be more accurately measured by using a wavelength range in which a signal indicating the absorbance of the coloring component is strong and the ratio of the absorbance due to light absorption of hemoglobin to the total absorbance is small as much as possible. Therefore, it is preferable to set the measurement wavelength up to around 650 nm that is a little longer than around 600 nm that is the peak wavelength in the absorbance spectrum of the coloring component. More specifically, the measurement wavelength is preferably in a range

of 600 nm to 680 nm, more preferably in a range of 600 nm to 670 nm, and particularly preferably 605 nm as in the present embodiment.

Furthermore, in the present embodiment, the color-developing reagent in which the full width at half maximum of the peak wavelength region in the absorbance spectrum of the coloring component is about 500 nm to about 700 nm is used, but a color-developing reagent in which the full width at half maximum of the peak wavelength region is out of this range may be used. Note that, as described above, in consideration of light absorption characteristics of hemoglobin, desirably, the wavelength region in which the absorbance due to light absorption of hemoglobin is large (that is, 600 nm or less) and the measurement wavelength in the absorbance spectrum of the coloring component do not overlap with each other.

Hereinafter, a method for estimating the absorbance of the coloring component at a measurement wavelength of 605 nm in the present embodiment will be described. The component measurement device 1 measures the absorbance of the mixture X at four measurement wavelengths, that is, the second predetermined wavelength A2 to the fifth predetermined wavelength λ5 that are different from the measurement wavelength (605 nm), corrects the first measured value D1 of the absorbance of the mixture X at the measurement wavelength using the four measured values including the second measured value D2 to the fifth measured value D5 and the correction coefficient data 86, and estimates the absorbance of the coloring component at the measurement wavelength. The measurement wavelength in the present embodiment is the above-described first predetermined wavelength λ1.

Specifically, the component measurement device 1 uses, as the above-described four measured values including the second measured value D2 to the fifth measured value D5, the two measured values including the second measured value D2 and the third measured value D3 of the absorbance of the mixture X at the two predetermined wavelengths including the second predetermined wavelength λ2 and the third predetermined wavelength λ3 on a longer wavelength side than the first predetermined wavelength λ1 as the measurement wavelength, and the two measured values including the fourth measured value D4 and the fifth measured value D5 of the absorbance of the mixture X at the two predetermined wavelengths including the fourth predetermined wavelength A4 and the fifth predetermined wavelength λ5 on a shorter wavelength side than the first predetermined wavelength λ1 as the measurement wavelength.

More specifically, the component measurement device 1 uses, as the above-described four measured values including the second measured value D2 to the fifth measured value D5, two measured values including the second measured value D2 and the third measured value D3 of the absorbance of the mixture X at the two predetermined wavelengths including the second predetermined wavelength λ2 and the third predetermined wavelength A3 belonging to a wavelength region in which the influence of light scattering due to the hemocyte component or the like is dominant in the total absorbance on a longer wavelength side than the first predetermined wavelength λ1 as the measurement wavelength, and two measured values including the fourth measured value D4 and the fifth measured value D5 of the absorbance of the mixture X at the two predetermined wavelengths including the fourth predetermined wavelength A4 and the fifth predetermined wavelength λ5 belonging to a wavelength region in which the influence of light absorption due to hemoglobin is large in the total absorbance on a shorter wavelength side than the first predetermined wavelength λ1 as the measurement wavelength.

In other words, the component measurement device 1 uses, as the above-described second measured value D2 and third measured value D3, absorbances of the mixture X at the second predetermined wavelength λ2 and the third predetermined wavelength λ3, respectively, belonging to a longer wavelength region than the measurement wavelength belonging to the wavelength range (in the present embodiment, a wavelength range of 500 to 700 nm) corresponding to the full width at half maximum of the peak wavelength region in the absorbance spectrum of the coloring component to be measured, for example, belonging to a long wavelength region W1 on a longer wavelength side than the wavelength range W3.

In addition, the component measurement device 1 uses, as the above-described fourth measured value D4 and fifth measured value D5, the fourth measured value D4 and the fifth measured value D5 that are absorbances of the mixture X at the fourth predetermined wavelengths λ4 and the fifth predetermined wavelength λ5, respectively, belonging to a shorter wavelength region than the measurement wavelength belonging to the wavelength range (500 to 700 nm) corresponding to the full width at half maximum of the peak wavelength region in the absorbance spectrum of the coloring component to be measured, for example, belonging to a short wavelength region W2 on a shorter wavelength side than the wavelength range W3.

The absorbance acquisition unit 78 of the component measurement device 1 acquires the above-described first measured value D1 to fifth measured value D5. Specifically, the mixture X is irradiated with an irradiation light beam including emission wavelengths of the first predetermined wavelength λ1 to the fifth predetermined wavelength λ5 from the first light source 67 to the fifth light source 68d of the light emitting unit 66, respectively. The light receiving unit 72 receives a transmission light beam that has passed through the mixture X among the irradiation light beams. Then, the arithmetic unit 60 calculates the absorbance of the mixture X at each of the wavelengths from a relationship between the irradiation light beam and the transmission light beam, and stores the first measured value D1 to the fifth measured value D5, which are absorbances of the mixture X at the wavelengths, respectively, in the memory 62 as the measured value data

85. In addition to the absorbance of the mixture X at each wavelength, the arithmetic unit 60 also stores, as the measured value data 85, measurement values of the amount of irradiation light and the amount of transmission light at each wavelength in the memory 62. These series of processes for generating the measured value data 85 may be repeatedly performed at predetermined time intervals. The predetermined time interval is, for example, an interval of 5 milliseconds, but is not limited thereto. The absorbance acquisition unit 78 of the component measurement device 1 can acquire the measured value data 85 from the memory 62. A means by which the absorbance acquisition unit 78 acquires the first measured value D1 to the fifth measured value D5 is not limited to the means described above, and the absorbance acquisition unit 78 can acquire the first measured value D1 to the fifth measured value D5 by various known means instead of the above-described means.

Then, the absorbance correction unit 83 of the component measurement device 1 corrects the first measured value D1 using the second measured value D2 to the fifth measured value D5, and estimates the absorbance of the coloring component at the first predetermined wavelength λ1 (605 nm in this example) that is the measurement wavelength.

In particular, as can be seen from Figs. 10 and 11, in the long wavelength region W1 in which light scattering of the hemocyte component or the like is dominant, the absorbance spectrum of the mixture X is substantially linear. Therefore, if the second measured value D2 that is the absorbance at the second predetermined wavelength λ2 and the third measured value D3 that is the absorbance at the third predetermined wavelength λ3 can be acquired, it is possible to estimate, to some extent, the absorbance caused by the disturbance factor (noise) other than the absorbance caused by the coloring component at the first predetermined wavelength λ1 that is the measurement wavelength by obtaining the slope between the second measured value D2 and the third measured value D3. The component measurement device 1 calculates the glucose concentration in blood in consideration of the ratio between deoxyhemoglobin and oxyhemoglobin in the red blood cells in addition to the optical characteristics due to the hemocyte component and the like in the blood. Therefore, the component measurement device 1 can perform correction with higher accuracy by using two wavelengths (the fourth predetermined wavelength and the fifth predetermined wavelength) selected according to the ratio between deoxyhemoglobin and oxyhemoglobin.

Specifically, as the fourth predetermined wavelength λ4, a wavelength at which the difference in absorption coefficient between deoxyhemoglobin and oxyhemoglobin is equal to or less than the first predetermined value is used, and as the fifth predetermined wavelength λ5, a wavelength at which the difference in absorption coefficient between deoxyhemoglobin and oxyhemoglobin is larger than the first predetermined value is used. More specifically, as the fourth predetermined wavelength λ4, a wavelength at which the ratio of an absorption coefficient of oxyhemoglobin to an absorption coefficient of deoxyhemoglobin (see Fig. 13) is equal to or more than the first threshold as the predetermined threshold is used, and as the fifth predetermined wavelength λ5, a wavelength at which the ratio of an absorption coefficient of oxyhemoglobin to an absorption coefficient of deoxyhemoglobin is less than the first threshold is used. In other words, the component measurement device 1 uses, as the fourth predetermined wavelength λ4 and the fifth predetermined wavelength λ5, two wavelengths, that is, a wavelength at which the ratio of an absorption coefficient of oxyhemoglobin to an absorption coefficient of deoxyhemoglobin is equal to or more than the first threshold and a wavelength at which the ratio is less than the first threshold. As a result, when correcting the first measured value D1 using the second measured value D2 to the fifth measured value D5, the absorbance correction unit 83 can perform correction with higher accuracy in consideration of the ratio between deoxyhemoglobin and oxyhemoglobin.

The two wavelengths selected according to the ratio between deoxyhemoglobin and oxyhemoglobin are preferably two wavelengths between which the difference in light absorption of hemoglobin due to the ratio between deoxyhemoglobin and oxyhemoglobin is large. Therefore, in the present embodiment, as the fourth predetermined wavelength λ4, a wavelength at which the ratio of an absorption coefficient of oxyhemoglobin to an absorption coefficient of deoxyhemoglobin is 0.8 or more, that is, a wavelength in a range of 520 nm to 550 nm, or a wavelength in a range of 565 nm to 585 nm is used. In addition, as the fifth predetermined wavelength λ5, it is preferable to use a wavelength at which the ratio of an absorption coefficient of oxyhemoglobin to an absorption coefficient of deoxyhemoglobin is less than 0.8, that is, a wavelength in a range of more than 550 nm and less than 565 nm, or a wavelength in a range of more than 585 nm and less than 600 nm. In the present embodiment, as the fourth predetermined wavelength λ4, a wavelength at which the absorption coefficient of deoxyhemoglobin is equal to the absorption coefficient of oxyhemoglobin, that is, in the present embodiment, a wavelength around 520 nm, around 545 nm, around 570 nm, or around 580 nm is preferably used so that the amount of the entire hemoglobin or the hematocrit value can be simultaneously estimated. In addition, the fifth predetermined wavelength λ5 is more preferably around 560 nm where the difference in absorption coefficient is the largest in a range of more than 550 nm and less than 565 nm, or 585 to 590 nm where the difference in absorption coefficient is the largest in a range of more than 585 nm and 600 nm or less.

As described above, in the short wavelength region W2 in which the light absorption of the entire hemoglobin greatly varies depending on the ratio between deoxyhemoglobin and oxyhemoglobin, by using the fourth predetermined wavelength λ4 and the fifth predetermined wavelength λ5 between which the difference in light absorption of the entire hemoglobin is large, the absorbance of noise at the first predetermined wavelength λ1 (605 nm in the present embodiment), which is the measurement wavelength, can be accurately estimated in consideration of the ratio between deoxyhemoglobin and oxyhemoglobin. Therefore, the component measurement device 1 can accurately perform the measurement of the absorbance of the coloring component at the first predetermined wavelength λ1 that is the measurement wavelength, and further the measurement of the measurement target component (in the present embodiment, glucose concentration measurement).

In the present embodiment, only the fourth predetermined wavelength A4 and the fifth predetermined wavelength λ5 are wavelengths in consideration of the influence of the ratio between deoxyhemoglobin and oxyhemoglobin, but the present invention is not limited thereto. In addition to the fourth predetermined wavelength A4 and the fifth predetermined wavelength λ5, the second predetermined wavelength λ2 and the third predetermined wavelength A3 may also be wavelengths in consideration of the influence of the ratio between deoxyhemoglobin and oxyhemoglobin.

Specifically, as the third predetermined wavelength λ3 in the long wavelength region W1 in which light scattering due to a hemocyte component or the like is dominant, a wavelength at which the difference in absorption coefficient between deoxyhemoglobin and oxyhemoglobin is equal to or less than a second predetermined value is used, and similarly, as the second predetermined wavelength λ2 in the long wavelength region W1, a wavelength at which the difference in absorption coefficient between deoxyhemoglobin and oxyhemoglobin is larger than the second predetermined value is used. More specifically, it is preferable to use, as the third predetermined wavelength λ3, a wavelength at which the ratio of an absorption coefficient of oxyhemoglobin to an absorption coefficient of deoxyhemoglobin is equal to or more than the above-described first threshold and equal to or less than a second threshold, and to similarly use, as the second predetermined wavelength A2 in the long wavelength region W1, a wavelength at which the ratio of an absorption coefficient of oxyhemoglobin to an absorption coefficient of deoxyhemoglobin is less than the above-described first threshold or a wavelength at which the ratio is larger than the second threshold. Here, the second threshold is a predetermined threshold different from the first threshold and larger than the first threshold. That is, as the second predetermined wavelength A2 and the third predetermined wavelength λ3, it is preferable to use two wavelengths at which the ratios of an absorption coefficient of oxyhemoglobin to an absorption coefficient of deoxyhemoglobin are in different ranges. As a result, when correcting the first measured value D1 using the second measured value D2 to the fifth measured value D5, the absorbance correction unit 83 can perform correction with high accuracy in further consideration of the ratio between deoxyhemoglobin and oxyhemoglobin.

In particular, in the long wavelength region W1, although the influence of light scattering due to the hemocyte component or the like is dominant, the influence of light absorption due to hemoglobin is also included to the same extent as in the measurement wavelength of the measurement target component. Thus, it is preferable to use, as the second predetermined wavelength λ2 and the third predetermined wavelength λ3, two wavelengths between which the light absorption due to hemoglobin relatively greatly varies depending on the ratio between deoxyhemoglobin and oxyhemoglobin.

Therefore, in the present embodiment, it is preferable to use, as the third predetermined wavelength λ3, a wavelength in a range in which the ratio of an absorption coefficient of deoxyhemoglobin is relatively close to an absorption coefficient of oxyhemoglobin, that is, a wavelength in a range of 0.8 or more and 1.56 or less, and it is more preferable to use a wavelength in a range of 790 nm to 860 nm. Furthermore, it is also preferable that the third predetermined wavelength λ3 is in the long wavelength region W1 and is a wavelength at which the absorbance of the coloring component included in the total absorbance at the third predetermined wavelength A3 is 3% or less, and more preferably 1% or less of the absorbance of the coloring component included in the total absorbance at the measurement wavelength, that is, a wavelength of 725 to 790 nm. In other words, it is particularly preferable to use a wavelength equal to or longer than a wavelength at a tail of a long wavelength side of a peak wavelength region of the absorbance spectrum of the coloring component. As a result, an influence of light absorption of the coloring component can be eliminated, and noise in which an influence of light scattering due to a hemocyte component or the like in the long wavelength region W1 is dominant can be more accurately estimated. Note that the above-described "total absorbance" in "the absorbance of the coloring component included in the total absorbance" means the absorbance of the whole mixture. The "absorbance of the coloring component" in the "absorbance of the coloring component included in the total absorbance" means an absorbance of a reaction product generated by a coloring reaction between the measurement target component in blood and the color-developing reagent in the reagent, that is, an absorbance derived from the coloring component in the mixture.

Further, as the second predetermined wavelength λ2, a wavelength in the long wavelength region W1 and is a wavelength at which the absorbance of the coloring component included in the total absorbance at the second predetermined wavelength A2 is 10% or less, and preferably 6% or less of the absorbance of the coloring component included in the total absorbance at the measurement wavelength and in a range of 725 nm or more and less than 790 nm is used, and the absorbance of the coloring component included in the total absorbance at the second predetermined wavelength λ2 is more preferably 1% or less, and still more preferably substantially 0%. That is, as the second predetermined wavelength λ2, it is more preferable to use a wavelength that is longer than 860 nm and less than 950 nm, and in this range, it is particularly preferable to use, as the second predetermined wavelength λ2, a wavelength of 940 nm to 950 nm at which the ratio between deoxyhemoglobin and oxyhemoglobin is larger than that at the third

predetermined wavelength.

As described above, the component measurement device 1 can correct the first measured value D1, which is the measured value of absorbance of the mixture X at the measurement wavelength, using the second measured value D2 to the fifth measured value D5, which are the measured values of absorbances of the mixture X at the second predetermined wavelength A2 to the fifth predetermined wavelength λ5, respectively, and can estimate the absorbance of the coloring component at the measurement wavelength.

Next, a method for correction by the absorbance correction unit 83 of the component measurement device 1 will be described.

As described above, the memory 62 of the component measurement device 1 stores the measured value data 85 of the first measured value D1 to the fifth measured value D5 that are absorbances of the mixture X at the first predetermined wavelength λ1 to the fifth predetermined wavelength λ5, respectively, and measured by the measurement optical system 64, a group of correction coefficient data 86 correlated with the absorbance of the mixture X at each of the second predetermined wavelength λ2 to the fifth predetermined wavelength A5, and the calibration curve data 90 indicating the relationship between the absorbance of the coloring component in the mixture X obtained by correcting, using the correction coefficient data 86, the absorbance of the mixture X measured at the first predetermined wavelength λ1 and various physical quantities.

The absorbance correction unit 83 calculates the absorbance of the coloring component at the first predetermined wavelength λ1 that is the measurement wavelength on the basis of the measured value data 85 and the correction coefficient data 86 stored in the memory 62.

Here, the correction coefficient data 86 is derived by regression analysis performed in advance using the following formula (2).

[Math. 1]

$$B(\lambda 1) = b0 + b1 * B(\lambda 2) + b2 * B(\lambda 3) + b3 * B(\lambda 4) + b4 * B(\lambda 5) \qquad (2)$$

B(λ) means an absorbance caused by a disturbance factor (noise) other than the absorbance of the coloring component at the wavelength λ, and regression calculation is performed using various types of blood specimens according to the formula (2) to derive coefficients b0, b1, b2, b3, and b4. Specifically, in the present embodiment, on the basis of the above-described selection standard for the second predetermined wavelength A2 to the fifth predetermined wavelength A5, 940 nm is used as the second predetermined wavelength A2, 855 nm is used as the third predetermined wavelength A3, 520 nm is used as the fourth predetermined wavelength A4, and 589 nm is used as the fifth predetermined wavelength A5. Note that these wavelengths are the median values for the light sources used, and the actual wavelength width includes an individual difference of about ±5 to 10 nm with respect to the median values. In addition, as for the various types of blood specimens, basically six blood specimens having different component compositions and each having a hematocrit value adjusted in a range of 10% to 70% are prepared, absorbance spectra of the adjusted blood specimens are measured, and the coefficients b0, b1, b2, b3, and b4 are derived using regression analysis. The number of observations performed this time is 766 in total. On the basis of these derived coefficients b0 to b4, a group of correction coefficients correlated with the absorbances of the mixture X at the second predetermined wavelength λ2 to the fifth predetermined wavelength λ5, respectively, is derived. By using the correction coefficient data 86 including the correction coefficients, the measured value of absorbance of the mixture X at 605 nm that is the measurement wavelength can be corrected from the measured values of absorbances of the mixture X at 520 nm, 589 nm, 855 nm, and 940 nm, and the absorbance of the coloring component at 605 nm can be estimated.

Here, each of the coefficients b0 to b4 obtained by the regression calculation can be determined as a value unique to the measurement system, and does not vary depending on the hematocrit value. Therefore, the numerical values (measured values) of B(λ2) to B(λ5) used for regression calculation vary depending on the hematocrit value.

Fig. 14 is a graph illustrating the degree of influence by the measured value in the long wavelength region W1 (indicated as "W1" in Fig. 14) and the degree of influence by the measured value in the short wavelength region W2 (indicated as "W2" in Fig. 14) in the noise amount (hereinafter also simply referred to as "noise absorbance"), which is the absorbance caused by the disturbance factor (noise) other than the coloring component at the measurement wavelength in the above-described regression calculation. The term "degree of influence" as used herein means an occupancy of data. As illustrated in Fig. 14, considering the results of the measured data obtained by the above-described regression calculation, when the noise absorbance at the first predetermined wavelength λ1, which is the measurement wavelength, is estimated using the second measured value D2 to the fifth measured value D5, the second measured value D2 and the third measured value D3 at the second predetermined wavelength A2 and the third predetermined wavelength λ3 in the long wavelength region W1 are decreased in the degree of influence from 92% to 90% as the hematocrit value increases from 10% to 70% (see "W1" in Fig. 14). Meanwhile, the fourth measured value D4 and the fifth measured value D5 at the fourth predetermined wavelength λ4 and the fifth predetermined wavelength λ5 in the short wavelength region W2 are

increased in the degree of influence from 8% to 10% as the hematocrit value increases from 10% to 70% (see "W2" in Fig. 14). As described above, since the degrees of influence in the long wavelength region W1 and the short wavelength region W2 used vary depending on the hematocrit value, the noise absorbance at the measurement wavelength can be more accurately estimated, and as a result, the absorbance of the coloring component at the measurement wavelength can be more accurately estimated. When the second measured value D2 to the fifth measured value D5 include absorption of the coloring component, correction calculation needs to be performed on the second measured value D2 to the fifth measured value D5 to calculate B(A) that is noise absorbance.

When the component measurement device 1 uses, as the fourth predetermined wavelength A4, a wavelength at which the absorption coefficient of deoxyhemoglobin is equal to the absorption coefficient of oxyhemoglobin (that is, 520 nm, 545 nm, 570 nm, or 580 nm in Fig. 12) in the short wavelength region W2 in which the influence of light absorption due to hemoglobin is overwhelmingly large, the hematocrit value can be calculated from the fourth measured value D4, or from the fourth measured value D4 and the second measured value D2 using a wavelength at which the absorption coefficient of deoxyhemoglobin is equal to the absorption coefficient of oxyhemoglobin (800 nm in Fig. 12) in the long wavelength region W1 in which the influence of light scattering due to the hemocyte component or the like is large. The hematocrit value can be calculated from the calibration curve of the absorbance of hemoglobin and the hematocrit value stored in the memory 62.

Referring to Fig. 7, the measurement target component calculation unit 84 of the component measurement device 1 calculates the measurement target component (glucose concentration in the present embodiment) in the blood based on the first measured value D1 to the fifth measured value D5 acquired by the absorbance acquisition unit 78 and the absorbance of the coloring component at the first predetermined wavelength $\lambda 1$ as the measurement wavelength estimated by the absorbance correction unit 83.

Specifically, the measurement target component calculation unit 84 calculates the hematocrit value using at least one of the first measured value D1 to the fifth measured value D5 acquired by the absorbance acquisition unit 78. In the present embodiment, the measurement target component calculation unit 84 estimates the absorbance of hemoglobin from the fourth measured value D4 or from the fourth measured value D4 and the second measured value D2, and calculates the hematocrit value using the calibration curve indicating the relationship between the absorbance of hemoglobin in the mixture X and the hematocrit value stored in the memory 62. Here, when the fourth measured value D4 or the fourth measured value D4 and the second measured value D2 include absorption of the coloring component, the measurement target component calculation unit 84 may calculate the hematocrit value from a correction value acquired by performing correction calculation of subtracting an absorption component of the coloring component from the fourth measured value D4 or from the fourth measured value D4 and the second measured value D2.

Then, the measurement target component calculation unit 84 calculates the concentration of glucose as the measurement target component from the absorbance of the coloring component at the first predetermined wavelength $\lambda 1$ that is the measurement wavelength and the calculated hematocrit value using the calibration curve indicating a relationship with the measurement target component stored in the memory 62.

Furthermore, in the present embodiment, the measurement target component calculation unit 84 is configured to correct the measured value of the measurement target component calculated as described above on the basis of the absorption information of hemoglobin contained in the blood.

Hereinafter, a method of correction for the measurement target component by the measurement target component calculation unit 84 of the component measurement device 1 will be described.

As described above, in improving the estimation accuracy of the absorbance derived from the coloring component to be measured, the absorption information of hemoglobin contained in the blood specimen is important. However, as for the absorption information of hemoglobin, the oxygen saturation, that is, the ratio between deoxyhemoglobin and oxyhemoglobin is different between blood specimens. For example, arterial blood has high oxygen saturation, whereas venous blood has low oxygen saturation. Since the ratio between deoxyhemoglobin and oxyhemoglobin varies according to the variation in the oxygen saturation, the correction based on the absorption of hemoglobin can be affected by the variation. Therefore, in the present embodiment, the measurement target component calculation unit 84 corrects the measured value of the measurement target component in consideration of the influence caused by the difference in oxygen saturation in the blood specimen. Since the correction is performed in consideration of the difference in oxygen saturation between blood specimens in this way, the measurement accuracy for the measurement target component in the blood (in the present embodiment, the concentration measurement accuracy for glucose) can be further improved.

As described above with reference to Fig. 12, with use of the fact that the absorption coefficient of deoxyhemoglobin and the absorption coefficient of oxyhemoglobin vary depending on the wavelength, in the present embodiment, the measurement target component calculation unit 84 is configured to estimate the oxygen saturation on the basis of the transmission light beam that is emitted when the blood is irradiated with the irradiation light beam having the wavelength at which the absorption coefficient of deoxyhemoglobin is equal to the absorption coefficient of oxyhemoglobin and the irradiation light beam having the wavelength at which the absorption coefficient of deoxyhemoglobin is different from the

absorption coefficient of oxyhemoglobin. In the following description, a wavelength at which the absorption coefficient of deoxyhemoglobin is equal to the absorption coefficient of oxyhemoglobin is also referred to as an "equivalent wavelength", and a wavelength at which the absorption coefficient of deoxyhemoglobin is different from the absorption coefficient of oxyhemoglobin is also referred to as a "non-equivalent wavelength". The equivalent wavelength is a wavelength at which the absorption coefficient of deoxyhemoglobin (Hb) and the absorption coefficient of oxyhemoglobin ($HbO_2$) intersect in Fig. 12, and examples thereof include 520 nm, 545 nm, 570 nm, and 580 nm. A wavelength in a range of ±10 nm, preferably ±5 nm of the above-mentioned wavelengths as median values can be regarded as an equivalent wavelength.

In the present embodiment, the fourth light source 68c is used as the first correction light source that emits the irradiation light beam having the equivalent wavelength, and the first light source 67 is used as the second correction light source that emits the irradiation light beam having the non-equivalent wavelength. As a result, as compared with a case where the component measurement device 1 is provided with the first correction light source and the second correction light source separately from the first light source 67 to the fifth light source 68d, the number of components of the component measurement device 1 can be reduced, and cost reduction and miniaturization can be realized. For example, the fourth predetermined wavelength λ4 of the fourth light source 68c may be 520 nm, and the first predetermined wavelength λ1 of the first light source 67 may be 605 nm. Alternatively, the fourth predetermined wavelength A4 of the fourth light source 68c may be 545 nm, and the first predetermined wavelength λ1 of the first light source 67 may be 560 nm. As a result, a green LED or an orange LED having high versatility can be used as the fourth light source 68c and the first light source 67, and the system can be constructed at low cost. However, the fourth predetermined wavelength A4 and the first predetermined wavelength λ1 may be any combination of wavelengths that satisfy the above-described conditions in the present disclosure. As the first correction light source and the second correction light source, the second light source 68a and the third light source 68b, or the fourth light source 68c and the fifth light source 68d may be used, or a light source different from the first light source 67 to the fifth light source 68d may be provided in the component measurement device 1.

A method of correction for the measurement target component by the measurement target component calculation unit 84 according to the present embodiment will be described in more detail with reference to Figs. 15, 16, and 17. Fig. 15 is a schematic view schematically illustrating stages in which blood is developed on the component measurement chip 2. Fig. 16 is a schematic view schematically illustrating a change in absorbance accompanying development of blood on the component measurement chip 2. Fig. 17 is a flowchart illustrating a method of correction for a measurement target component according to an embodiment of the present disclosure.

As illustrated in Fig. 15, when a blood specimen is supplied from the supply portion 24 of the component measurement chip 2 to the flow path 23, the blood supplied to the flow path 23 of the component measurement chip 2 moves in the flow path 23 of the component measurement chip 2 toward the measurement reagent 22, and then the blood reacts with an enzyme, a color-developing reagent, a hemolysis reagent, and an oxidation reagent contained in the measurement reagent 22. At this time, it is preferable that the measurement of the oxygen saturation of the blood is performed after the blood flows into the flow path 23 of the component measurement chip 2 and a sufficient amount of blood reaches the measurement reagent 22, and before the reaction between the blood and the measurement reagent 22 is started ((B) immediately after end of blood development in the drawing). Therefore, in the present embodiment, the measurement target component calculation unit 84 determines whether or not it is before the start of the reaction between the measurement target component in the blood and the measurement reagent 22 by utilizing the fact that there is a time lag between the inflow of the blood into the flow path 23 of the component measurement chip 2 and the start of the reaction of the measurement reagent 22, and then acquires the absorbance necessary for estimating the oxygen saturation before the start of the reaction. At this time, the light received by the light receiving unit is a transmission light beam that has passed through the unreacted measurement reagent 22 and the unreacted blood.

Specifically, as illustrated in Fig. 17, in step S101, the measurement target component calculation unit 84 of the component measurement device 1 performs blood development start determination.

Any method can be adopted for the blood development start determination. As illustrated in Fig. 16, as blood is injected into the flow path 23, the amount of transmission light received by the light receiving unit 72 changes, and the absorbance changes. Therefore, for example, the measurement target component calculation unit 84 may determine that the blood development has started when there is a predetermined time-series change (first time-series change) in the transmission light beam from at least one of the first light source 67 to the fifth light source 68d (for example, the fourth light source 68c that is the first correction light source) using the measured value data 85 generated at predetermined time intervals. Specifically, the measurement target component calculation unit 84 may determine, with the ratio of the amount of transmission light to the amount of irradiation light of the fourth predetermined wavelength λ4 as a light amount change rate (%), in a case where (1) the light amount change rate increases/decreases by more than 5% consecutively twice, a time point before the above period and next to a period in which (2) the light amount change rate is less than 1.2% consecutively twice as the blood development start point. As a result, the measurement target component calculation unit 84 can specify the start point of blood development as illustrated in Fig. 16.

Referring to Fig. 17 again, when it is determined in step S101 that blood development has started, the measurement target component calculation unit 84 proceeds to step S102 and performs blood development end

determination.

Any method can be adopted for the blood development end determination. For example, the measurement target component calculation unit 84 may determine that the blood development has ended when there is a predetermined time-series change (second time-series change) in the absorbance at at least one of the first predetermined wavelength λ1 to the fifth predetermined wavelength λ5 (for example, the fourth light source 68c that is the first correction light source) included in the measured value data 85. Specifically, the measurement target component calculation unit 84 may determine, as the blood development end point, a time point when (1) the difference between the absorbance at the fourth predetermined wavelength A4 belonging to the visible region and the average value of the absorbances at the second predetermined wavelength λ2 and the third predetermined wavelength λ3 belonging to the infrared region reaches 0.1 or more, and then (2) the time-series change amount (for example, three times moving average) of the absorbance at the fourth predetermined wavelength λ4 reaches less than 0.005. As a result, the measurement target component calculation unit 84 can specify the end point of blood development as illustrated in Fig. 16.

Referring to Fig. 17 again, in step S103, the measurement target component calculation unit 84 calculates a supplementary absorbance ratio immediately after the end of blood development.

The supplementary absorbance ratio is a ratio between the absorbance at the equivalent wavelength and the absorbance at the non-equivalent wavelength, and is information having a strong correlation with the oxygen saturation of the blood specimen. Therefore, in the present embodiment, as the correction based on the oxygen saturation, the measurement target component is corrected using the supplementary absorbance ratio. The supplementary absorbance ratio is calculated from the absorbances at the second predetermined wavelength λ2 and the third predetermined wavelength λ3 belonging to the infrared region and the absorbances at the fourth predetermined wavelength λ4 and the first predetermined wavelength λ1 belonging to the visible region immediately after the end of blood development using the following formula (3).

R = (absorbance at λ1 - average value of absorbance at λ2 and absorbance at λ3) ÷ (absorbance at λ4 - average value of absorbance at λ2 and absorbance at λ3) (3)

In the formula, R is the supplementary absorbance ratio, and the fourth predetermined wavelength λ4 is an equivalent wavelength, and the first predetermined wavelength λ1 is a non-equivalent wavelength.

Any method can be employed for the calculation of the supplementary absorbance ratio immediately after the end of blood development. For example, the measurement target component calculation unit 84 calculates the supplementary absorbance ratio based on one or more measured values within a predetermined period from the blood development end point determined in step S102 and included in the measured value data 85. The predetermined period is, for example, a period until the reaction between the blood and the measurement reagent 22 starts after the blood is developed in the flow path 23 of the component measurement chip 2, and may be about 150 milliseconds. As an example, the measurement target component calculation unit 84 may calculate the supplementary absorbance ratio from each of measured values at five consecutive time points at an interval of 5 milliseconds from the blood development end point, and may use an average value thereof as the supplementary absorbance ratio of the blood specimen. In the calculation of the average value, when there is a value that is not included in a predetermined range with reference to the average value (for example, a range of ±0.05 of the average value), the measurement target component calculation unit 84 may calculate the supplementary absorbance ratio of the blood specimen excluding such a value as a singular point. As a result, the calculation accuracy of the supplementary absorbance ratio of the blood specimen is improved, and the measurement accuracy for the measurement target component in the blood is improved. In the calculation of the average value, when the number of values not included in the predetermined range with reference to the average value (for example, a range of ±0.05 of the average value) is equal to or larger than a predetermined number, the measurement target component calculation unit 84 does not have to correct the measurement target component by using the supplementary absorbance ratio according to the subsequent process. As a result, it possible to prevent, beforehand, the measurement accuracy for the measurement target component from deteriorating due to the correction using the supplementary absorbance ratio.

In step S104, the measurement target component calculation unit 84 corrects the measurement target component using the supplementary absorbance ratio.

Any method can be employed for the calculation of the supplementary absorbance ratio immediately after the end of blood development. For example, as a first method, the measurement target component calculation unit 84 may correct the concentration of glucose as the measurement target component by applying the supplementary absorbance ratio calculated in step S103 to the following formula (4).

$$Bg = Bg' - f(R) \quad (4)$$

In the formula, Bg is a corrected glucose concentration, Bg' is a glucose concentration before correction, R is a

supplementary absorbance ratio, and f(R) is a correction value, and is a function with the supplementary absorbance ratio R as an argument.

As described above, use of the first method can improve the measurement accuracy for the measurement target component in the blood (in the present embodiment, the concentration measurement accuracy for glucose) measured by the component measurement device 1 regardless of the oxygen saturation of the blood specimen.

As a second method, by utilizing the fact that the absorbance at the equivalent wavelength (in the present embodiment, the absorbance at the fourth predetermined wavelength λ4) has a strong correlation with the hemocyte amount, the measurement target component calculation unit 84 may correct the concentration of glucose as the measurement target component using a supplementary absorbance x that is based on the supplementary absorbance ratio and the information about the absorbance at the equivalent wavelength ("absorbance at λ4 - average value of absorbance at λ2 and absorbance at λ3" in the above formula (3)). Specifically, the measurement target component calculation unit 84 may correct the concentration of glucose as the measurement target component using the following formulae (5) and (6).

x = R × (absorbance at λ4 - average value of absorbance at λ2 and absorbance at λ3) = absorbance at λ1 - average value of absorbance at λ2 and absorbance at A3 (5)

$$Bg = Bg' - F(x) \quad (6)$$

In the formulae, R is a supplementary absorbance ratio, x is a supplementary absorbance, the fourth predetermined wavelength λ4 is an equivalent wavelength, the first predetermined wavelength λ1 is a non-equivalent wavelength, Bg is a corrected glucose concentration, Bg' is a glucose concentration before correction, F(x) is a correction value, and is a function with x as an argument.

As described above, use of the second method can improve the measurement accuracy for the measurement target component in the blood (in the present embodiment, the concentration measurement accuracy for glucose) measured by the component measurement device 1 regardless of the oxygen saturation and hemocyte amount of the blood specimen. As shown in the formula (5), the supplementary absorbance x correlated with the oxygen saturation and the hemocyte amount is information on the absorbance at the non-equivalent wavelength immediately after the end of blood development (absorbance at λ1 - average value of absorbance at λ2 and absorbance at λ3). Therefore, according to the second method, the correction can be performed with a smaller amount of calculation than in the first method in which both the information on the absorbance at the equivalent wavelength and the information on the absorbance at the non-equivalent wavelength are used.

In the present embodiment, the functions f(R) and f(x) may be constructed by a statistical method such as machine learning or deep learning. For example, the functions f(R) and f(x) may be constructed by a statistical method using measured values of absorbance and glucose concentration in experiments on various types of blood as training data. As a result, the correction accuracy for the glucose concentration can be improved by accumulating the training data. However, the functions f(R) and f(x) may include a predetermined arithmetic expression that is not constructed by a statistical method.

As described above, through the processes of steps S101 to S104, the measurement target component calculation unit 84 can determine whether or not a reaction between the measurement target component and the reagent is started based on a time-series change in the transmission light beam emitted from at least one of the two correction light sources (the fourth light source 68c as the first correction light source and the fifth light source 68d as the second correction light source) and received by the light receiving unit, estimate the oxygen saturation of the blood based on the transmission light beam emitted from the two correction light sources and received by the light receiving unit 72 before start of the reaction, and correct the measured value of the measurement target component based on the oxygen saturation.

Finally, a component measurement method executed by the above-described component measurement device 1 will be collectively described with reference to Fig. 18. Fig. 18 is a flowchart illustrating an example of a component measurement method executed by the component measurement device 1.

As illustrated in Fig. 18, the component measurement method according to the present disclosure includes: step S1 of acquiring a first measured value D1 that is an absorbance of a mixture X at a first predetermined wavelength λ1 as a measurement wavelength, a second measured value D2 that is an absorbance of the mixture X at a second predetermined wavelength λ2, a third measured value D3 that is an absorbance of the mixture X at a third predetermined wavelength λ3, a fourth measured value D4 that is an absorbance of the mixture X at a fourth predetermined wavelength λ4, and a fifth measured value D5 that is an absorbance of the mixture X at a fifth predetermined wavelength λ5, step S2 of correcting the first measured value D1 using the second measured value D2 to the fifth measured value D5 and correction of a measurement target component obtained by regression calculation, and acquiring the absorbance of the coloring

component at the first predetermined wavelength λ1 as the measurement wavelength, step S3 of calculating the hematocrit value using at least one of the first measured value D1 to the fifth measured value D5, and step S4 of calculating the measurement target component in the blood from the absorbance of the coloring component at the first predetermined wavelength λ1 as the measurement wavelength and the calculated hematocrit value.

Specifically, in step S1, the absorbance acquisition unit 78 acquires the first measured value D1 to the fifth measured value D5 using the light emitting unit 66 and the light receiving unit 72 in the measurement optical system 64. In step S2, the absorbance correction unit 83 corrects the first measured value D1 using the second measured value D2 to the fifth measured value D5 and correction of the measurement target component obtained by the regression calculation, and estimates and acquires the absorbance of the coloring component at the measurement wavelength. In step S3, the measurement target component calculation unit 84 calculates the hematocrit value based on the fourth measured value D4 or based on the fourth measured value D4 and the second measured value D2. Then, in step S4, the measurement target component calculation unit 84 calculates the glucose concentration from the acquired absorbance of the coloring component at the first predetermined wavelength λ1 that is the measurement wavelength and the calculated hematocrit value using a calibration curve indicating a relationship with the glucose concentration. At this time, the measurement target component calculation unit 84 corrects the glucose concentration on the basis of the oxygen saturation of the blood calculated based on the first measured value D1 to the fourth measured value D4.

As described above, the component measurement device 1 according to the present embodiment is a component measurement device for measuring the measurement target component in the blood using a reagent that reacts with the measurement target component in the blood based on optical characteristics of a coloring component generated by a coloring reaction between the measurement target component and the reagent. The component measurement device 1 is configured to correct the measured value of the measurement target component based on the oxygen saturation of the blood.

With such a configuration, the component measurement device 1 can correct the measured value of the measurement target component in consideration of the fact that the oxygen saturation differs by the blood specimen, and consequently improve the measurement accuracy for the measurement target component in the blood. Therefore, the component measurement device 1 according to the present disclosure can further improve the measurement accuracy of whole blood measurement that is performed using an absorptiometry.

The present disclosure has been described with reference to the drawings and embodiments, and it should be noted that those skilled in the art can make various modifications and corrections on the basis of the present disclosure. Thus, it should be noted that these modifications and corrections fall within the scope of the present disclosure. For example, configurations, functions, and the like included in the embodiments can be rearranged so as not to be logically inconsistent. In addition, configurations, functions, and the like included in the embodiments can be used in combination with other embodiments, and a plurality of configurations, functions, and the like can be combined, divided, or partially omitted.

For example, in the above-described embodiments, the glucose concentration is measured as the measurement of glucose as the measurement target component, but the measurement target is not limited to the concentration, and another physical quantity may be measured. In addition, in the above-described embodiments, glucose in the plasma component is exemplified as the measurement target component in blood, but the measurement target component is not limited thereto. For example, cholesterol, saccharides, ketone bodies, uric acid, hormones, nucleic acids, antibodies, antigens, and the like in blood can also be used as the measurement target components. Therefore, the component measurement device is not limited to a blood glucose level measurement device.

In addition, for example, in the above-described embodiments, it has been described that the oxygen saturation of blood is estimated based on the transmission light beam emitted from the two correction light sources and received by the light receiving unit 72 before the start of the reaction between the measurement target component and the reagent, but the present invention is not limited thereto. The oxygen saturation of blood may be estimated based on the transmission light beam emitted from the two correction light sources and received by the light receiving unit 72 after the reaction between the measurement target component and the reagent is at least partially started.

For example, in the above-described embodiments, the light receiving unit 72 receives a transmission light beam that has passed through the component measurement chip 2, but may receive reflected light reflected from the component measurement chip 2.

In addition, for example, in the above-described embodiments, the blood glucose level in the whole blood is measured without the step of separating the blood, but the blood after filtration and partial removal of a hemocyte component, dust, or the like may be used as the measurement target. In the step of separating the blood, the whole blood without filtration may be used, and calculation may be performed separately in a measurement area in which the blood is reacted with the measurement reagent 22 and in a correction area in which correction is performed.

Industrial Applicability

The present disclosure relates to a component measurement device and a component measurement method.

Reference Signs List

| | |
|---|---|
| 1 | Component measurement device |
| 2 | Component measurement chip |
| 10 | Housing |
| 10a | Main body portion |
| 10b | Chip attachment portion |
| 11 | Display unit |
| 12 | Detachment lever |
| 13 | Power button |
| 14 | Operation button |
| 21 | Base member |
| 22 | Measurement reagent (reagent) |
| 23 | Flow path |
| 23a | Gap |
| 24 | Supply portion |
| 25 | Cover member |
| 26 | Eject pin |
| 60 | Arithmetic unit |
| 62 | Memory |
| 63 | Power circuit |
| 64 | Measurement optical system |
| 66 | Light emitting unit |
| 67 | First light source (second correction light source) |
| 68a | Second light source |
| 68b | Third light source |
| 68c | Fourth light source (first correction light source) |
| 68d | Fifth light source |
| 69a | First throttle portion |
| 69b | Second throttle portion |
| 70 | Light emission control circuit |
| 72 | Light receiving unit |
| 74 | Light reception control circuit |
| 76 | Measurement instruction unit |
| 77 | Concentration measurement unit |
| 78 | Absorbance acquisition unit |
| 80 | Holder member |
| 83 | Absorbance correction unit |
| 84 | Measurement target component correction unit |
| 85 | Measured value data |
| 86 | Correction coefficient data |
| 90 | Calibration curve data |
| 100 | Component measurement device set |
| A | Flow direction |
| B | Flow path width direction |
| C | Thickness direction |
| D1 | First measured value |
| D2 | Second measured value |
| D3 | Third measured value |
| D4 | Fourth measured value |
| D5 | Fifth measured value |
| S | Chip attachment space |

| | |
|---|---|
| SL1 to SL5 | Irradiation position from light source |
| T1 | Distance between light source and first throttle portion |
| T2 | Distance between light source and light receiving unit |
| T3 | Distance between mixture and first throttle portion |
| T4 | Distance between light source and second throttle portion |
| W1 | Long wavelength region |
| W2 | Short wavelength region |
| W3 | Wavelength range corresponding to full width at half maximum |
| X | Mixture |
| $\lambda 1$ | First predetermined wavelength |
| $\lambda 2$ | Second predetermined wavelength |
| $\lambda 3$ | Third predetermined wavelength |
| $\lambda 4$ | Fourth predetermined wavelength |
| $\lambda 5$ | Fifth predetermined wavelength |
| R | Supplementary absorbance ratio |
| Bg, Bg' | Glucose concentration |
| x | Supplementary absorbance |

**Claims**

**1.** A component measurement device for measuring a measurement target component in blood using a reagent that reacts with the measurement target component in the blood based on optical characteristics of a coloring component generated by a coloring reaction between the measurement target component and the reagent,
the component measurement device being configured to correct a measured value of the measurement target component based on an oxygen saturation of the blood.

**2.** The component measurement device according to claim 1, comprising:

two correction light sources including a first correction light source that emits an irradiation light beam having a wavelength at which an absorption coefficient of deoxyhemoglobin is equal to an absorption coefficient of oxyhemoglobin, and a second correction light source that emits an irradiation light beam having a wavelength at which an absorption coefficient of deoxyhemoglobin is different from an absorption coefficient of oxyhemoglobin; and
a light receiving unit that receives, of the irradiation light beams from the two correction light sources, a light beam that has passed through the blood as a transmission light beam,
the component measurement device being configured to estimate the oxygen saturation of the blood based on the transmission light beam emitted from the two correction light sources and received by the light receiving unit.

**3.** The component measurement device according to claim 2, being configured to determine whether or not a reaction between the measurement target component and the reagent is started based on a time-series change in the transmission light beam emitted from at least one of the two correction light sources and received by the light receiving unit, and
configured to estimate the oxygen saturation of the blood based on the transmission light beam emitted from the two correction light sources and received by the light receiving unit before start of the reaction.

**4.** The component measurement device according to claim 2 or 3, comprising:

a first light source that emits an irradiation light beam having a measurement wavelength within a wavelength range corresponding to a full width at half maximum of a peak wavelength region in an absorbance spectrum of the coloring component;
a second light source that emits an irradiation light beam having a second wavelength and a third light source that emits an irradiation light beam having a third wavelength, the second wavelength and the third wavelength belonging to a longer wavelength region than the measurement wavelength; and
a fourth light source that emits an irradiation light beam having a fourth wavelength and a fifth light source that emits an irradiation light beam having a fifth wavelength, the fourth wavelength and the fifth wavelength belonging to a shorter wavelength region than the measurement wavelength that belongs to the wavelength range corresponding to the full width at half maximum,
the component measurement device being configured to receive, by the light receiving unit, among the irradiation

light beams from the first light source to the fifth light source, a light beam that has passed through the blood as a transmission light beam, and

configured to measure the measurement target component in the blood using a measured value of absorbance of a mixture containing the blood, the reagent, and the coloring component at the measurement wavelength and measured values of absorbances of the mixture at the second wavelength to the fifth wavelength,

wherein the first correction light source is the fourth light source, and the second correction light source is the first light source.

**5.** The component measurement device according to any one of claims 1 to 3, wherein the reagent contains, in addition to an oxidoreductase that specifically reacts with the measurement target component and a color-developing reagent that causes coloring according to an amount of reaction between the measurement target component and the oxidoreductase, a hemolysis reagent that hemolyzes the blood and an oxidation reagent that oxidizes hemoglobin in the blood.

**6.** A component measurement method for measuring a measurement target component in blood using a reagent that reacts with the measurement target component in the blood based on optical characteristics of a coloring component generated by a coloring reaction between the measurement target component and the reagent, the component measurement method comprising:

correcting a measured value of the measurement target component based on an oxygen saturation of the blood, wherein

the oxygen saturation of the blood is estimated based on a transmission light beam that has passed through the blood and the reagent.

FIG. 1

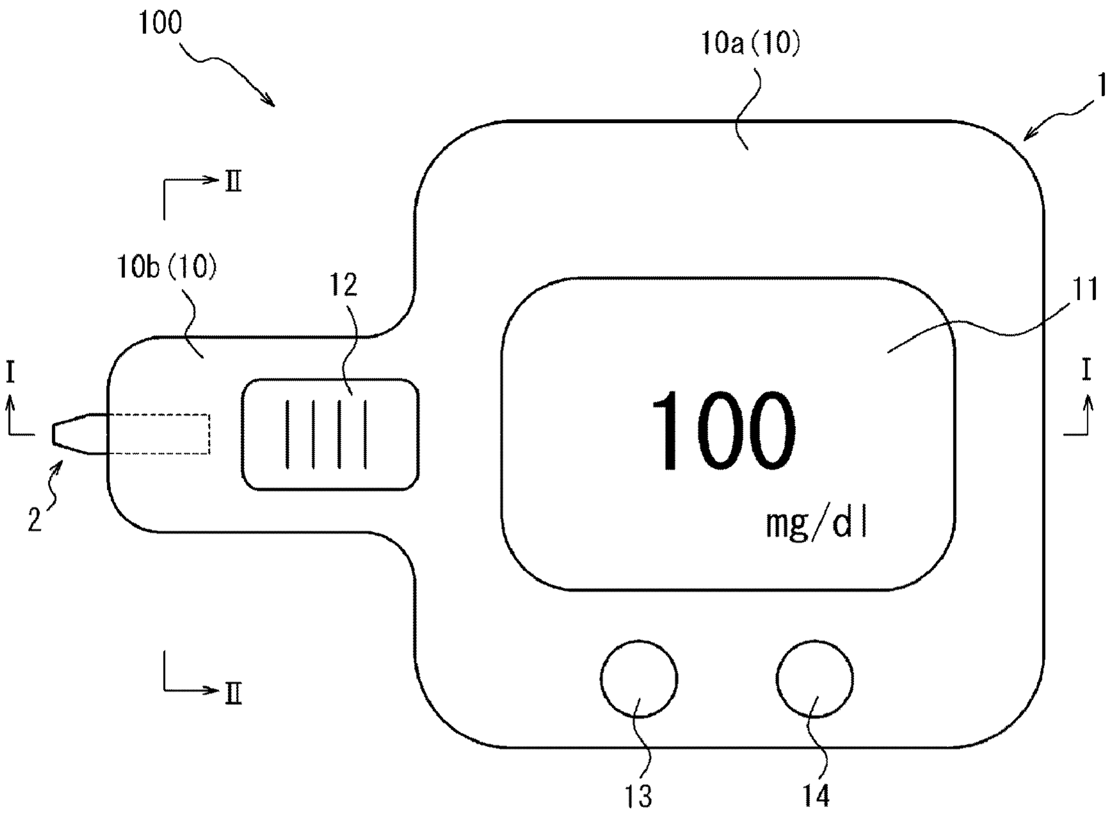

FIG. 2

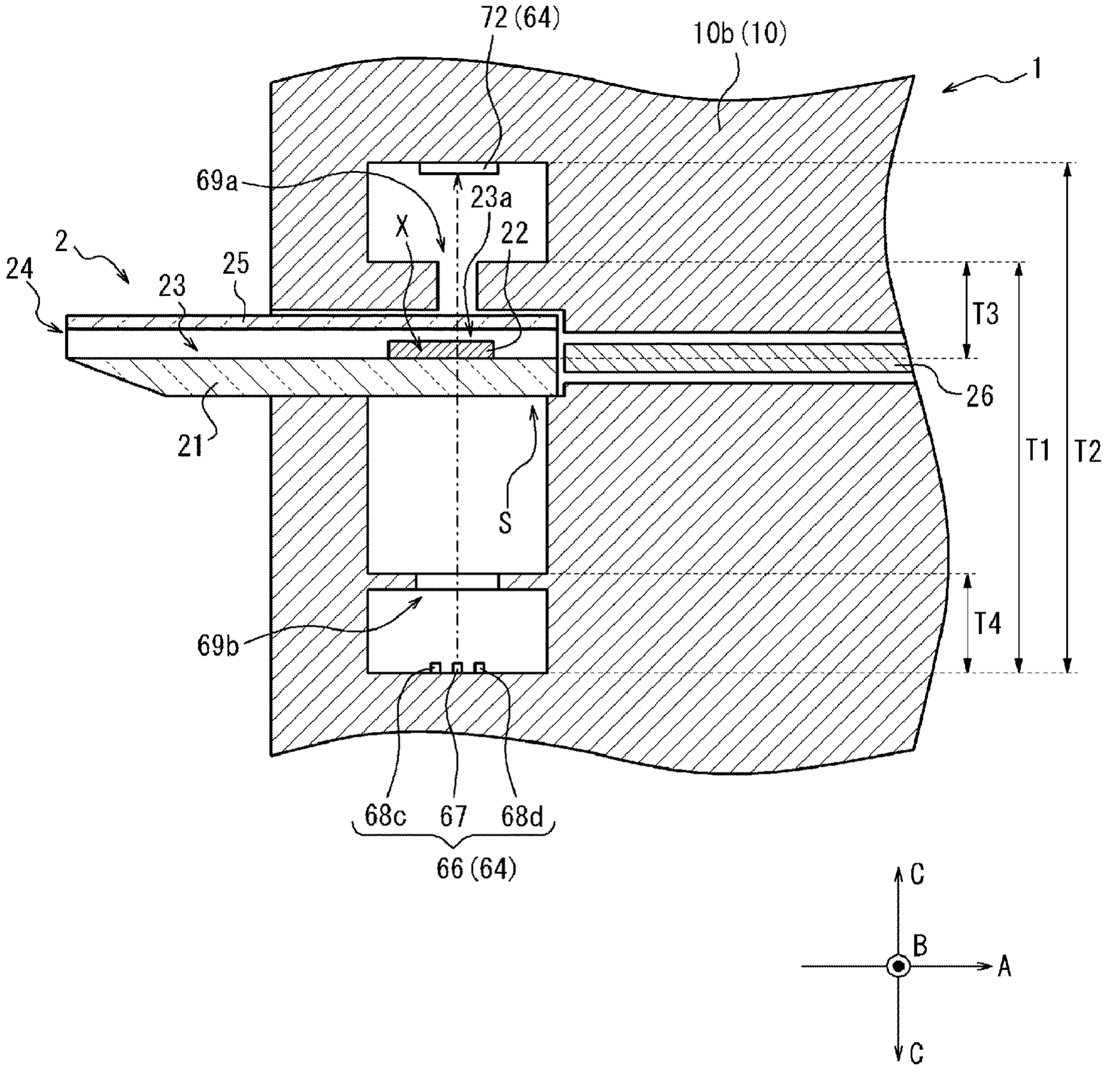
72 (64)
10b (10)
1
69a
23a
X
22
2
24
23
25
T3
26
T1
T2
21
S
T4
69b
68c
67
68d
66 (64)
C
B
A
C

FIG. 3

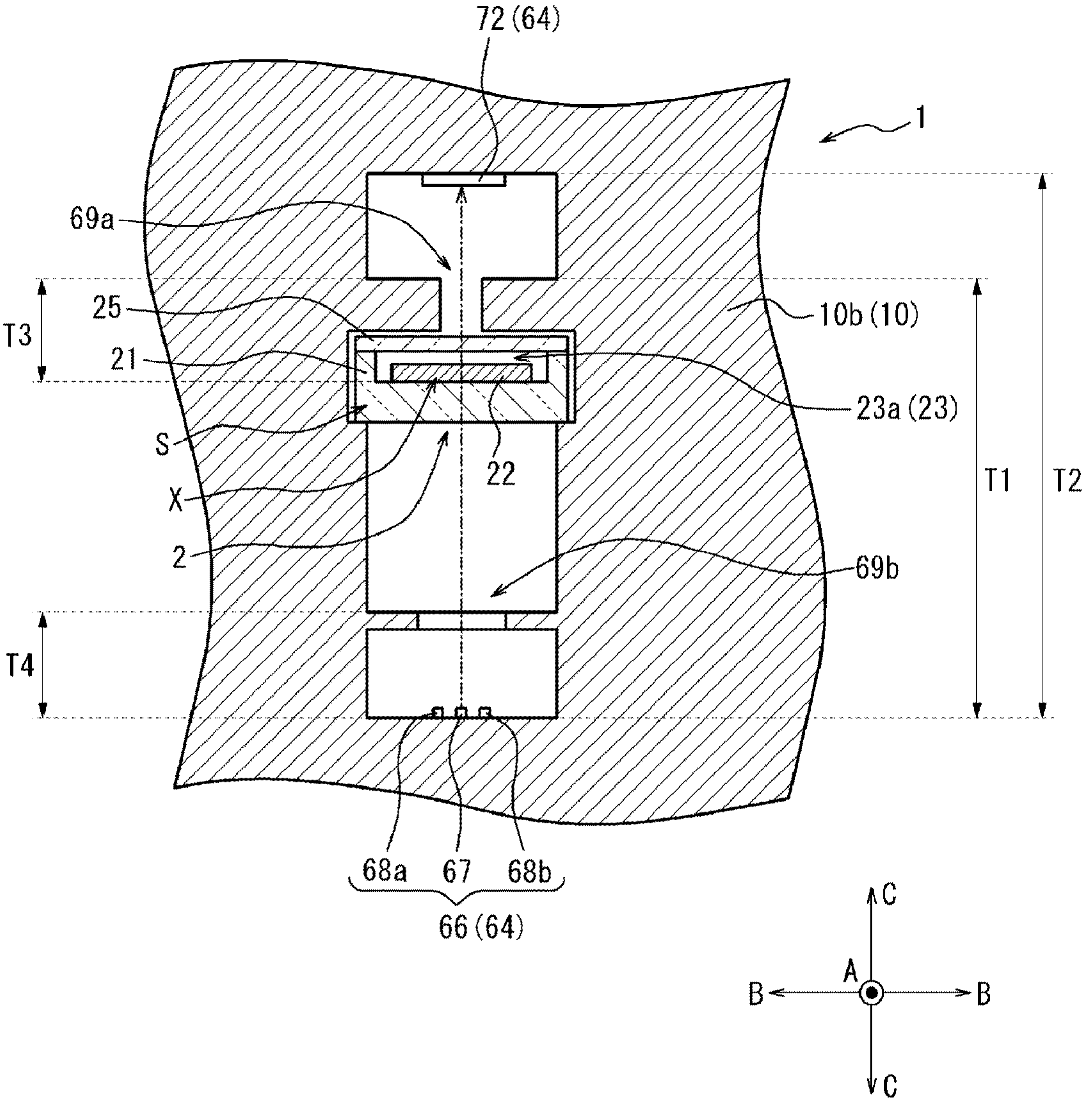
72 (64)
1
69a
T3
25
21
S
X
2
10b (10)
23a (23)
22
T1
T2
69b
T4
68a
67
68b
66 (64)
C
A
B
B
C

FIG. 4

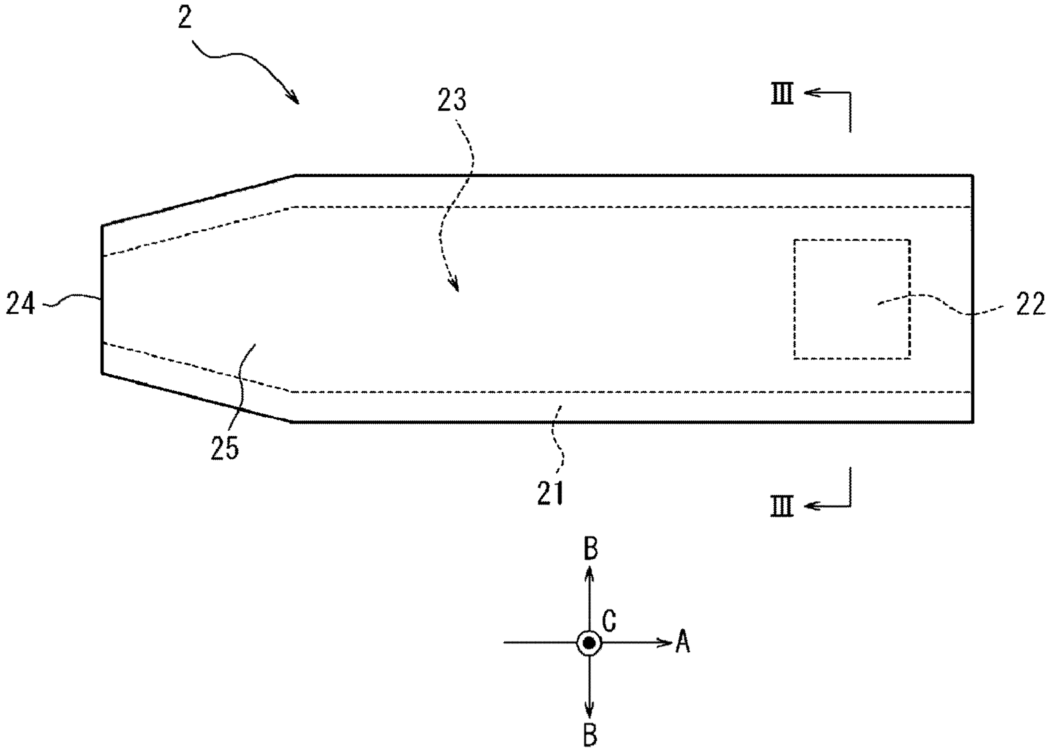


FIG. 5

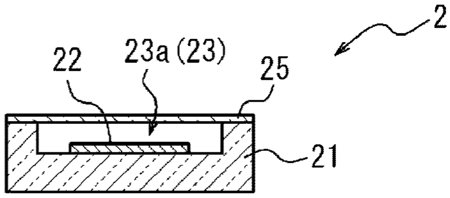

FIG. 6

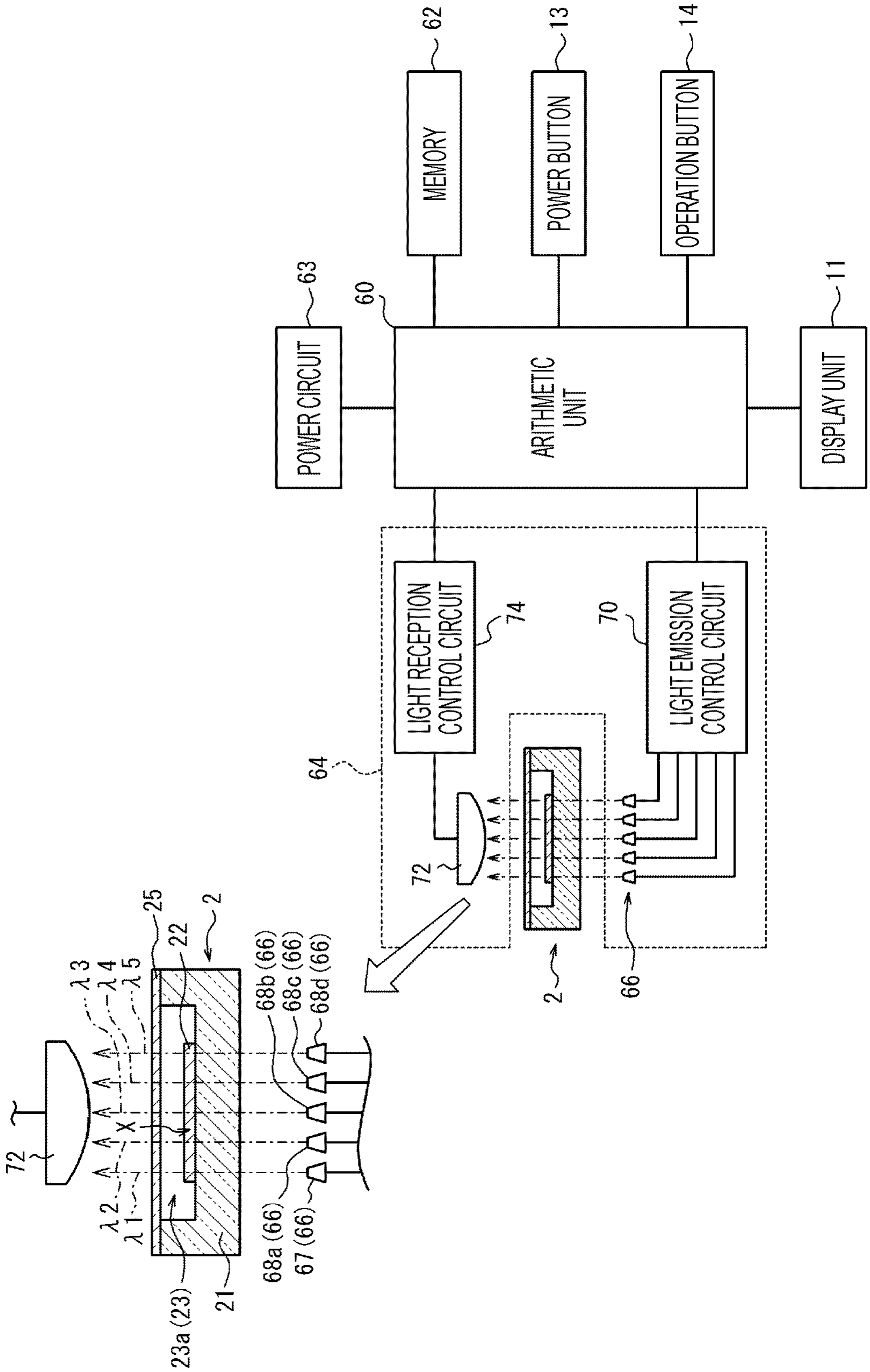

MEMORY
POWER BUTTON
OPERATION BUTTON
POWER CIRCUIT
ARITHMETIC UNIT
DISPLAY UNIT
LIGHT RECEPTION CONTROL CIRCUIT
LIGHT EMISSION CONTROL CIRCUIT
62
13
14
63
60
11
74
70
64
72
2
66
25
22
λ3
λ4
λ5
λ2
λ1
X
68b (66)
68c (66)
68d (66)
68a (66)
67 (66)
23a (23)
21

FIG. 7

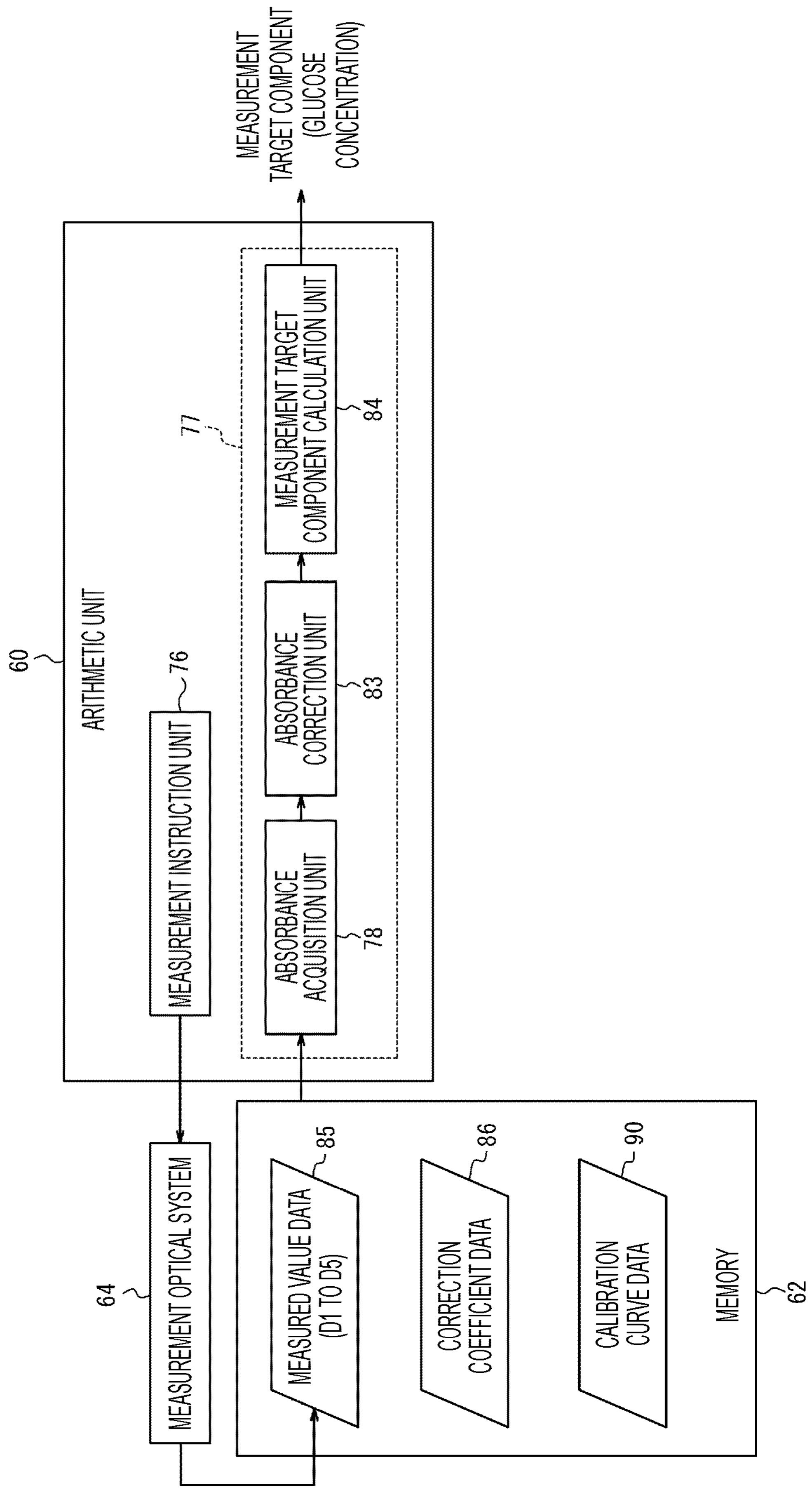
MEASUREMENT TARGET COMPONENT (GLUCOSE CONCENTRATION)
60
ARITHMETIC UNIT
76
MEASUREMENT INSTRUCTION UNIT
77
ABSORBANCE ACQUISITION UNIT
78
ABSORBANCE CORRECTION UNIT
83
MEASUREMENT TARGET COMPONENT CALCULATION UNIT
84
64
MEASUREMENT OPTICAL SYSTEM
85
MEASURED VALUE DATA (D1 TO D5)
86
CORRECTION COEFFICIENT DATA
90
CALIBRATION CURVE DATA
MEMORY
62

FIG. 8

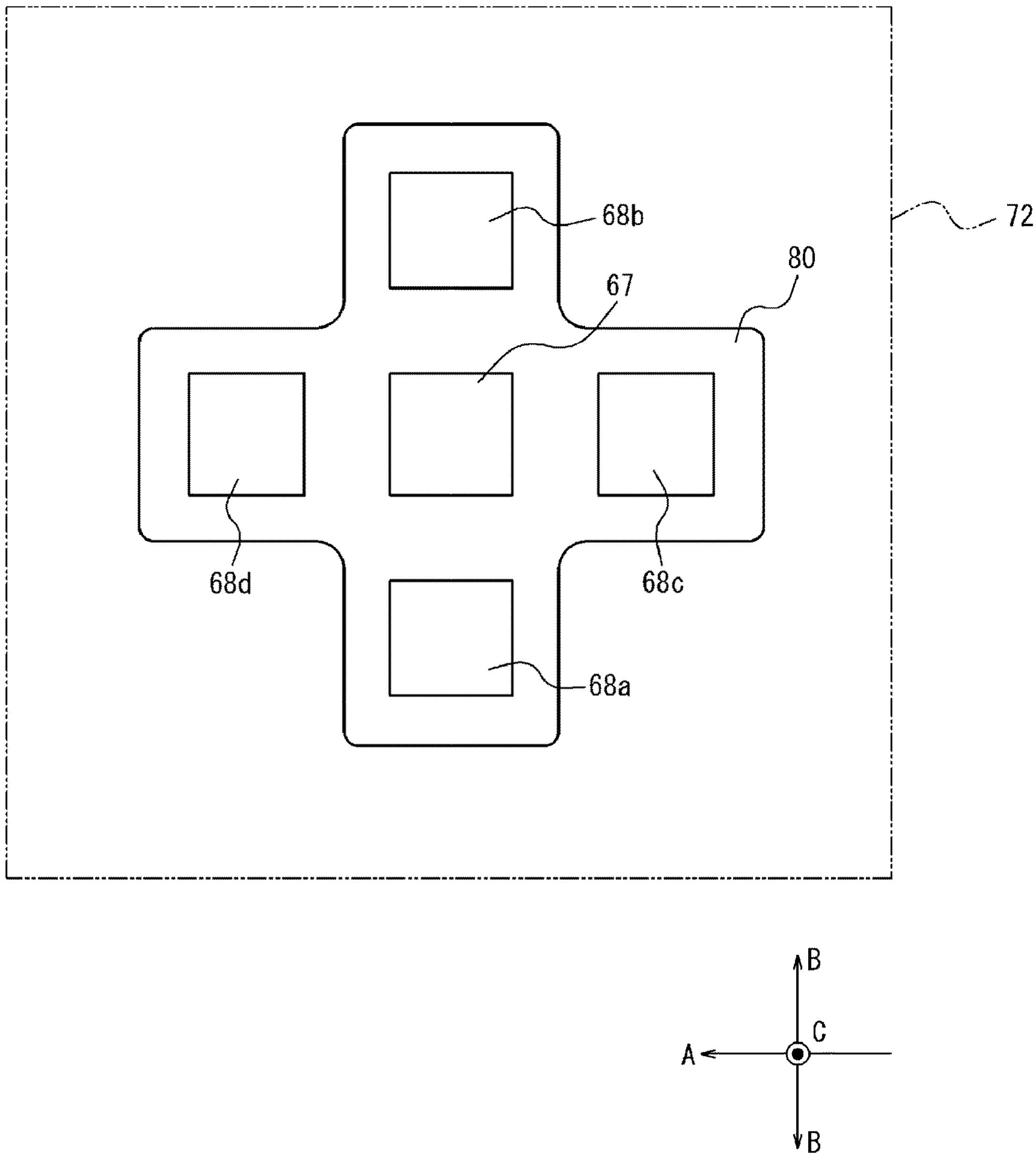

FIG. 9

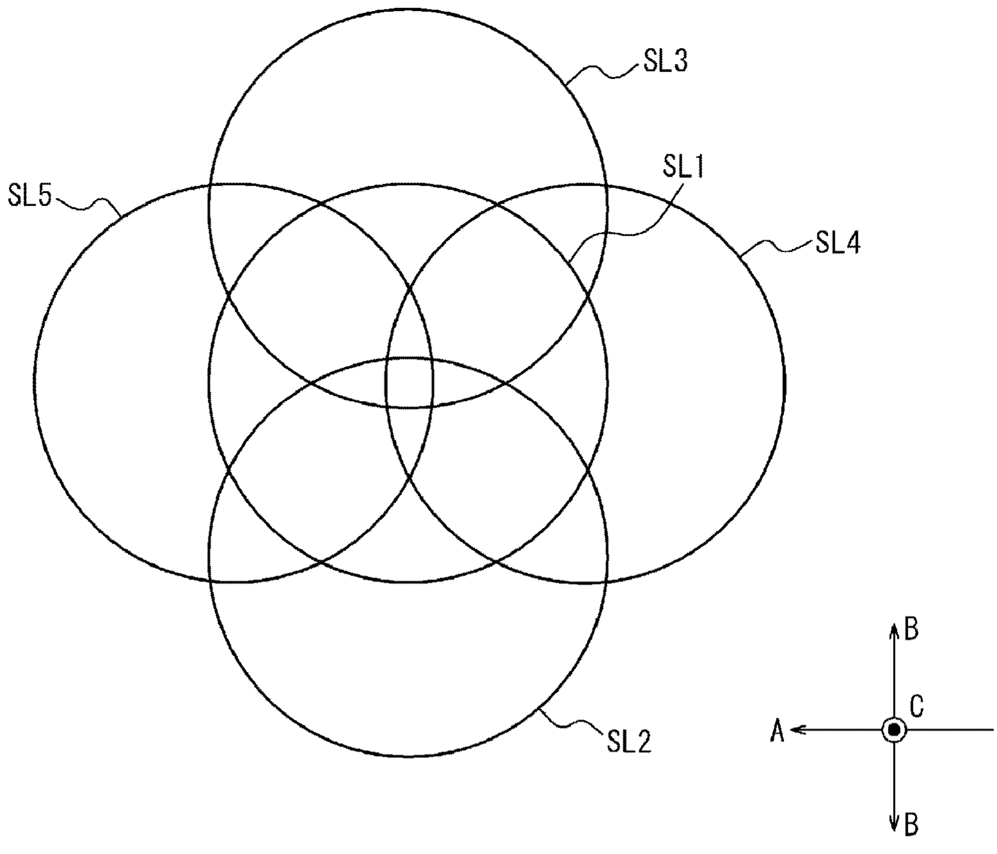

FIG. 10

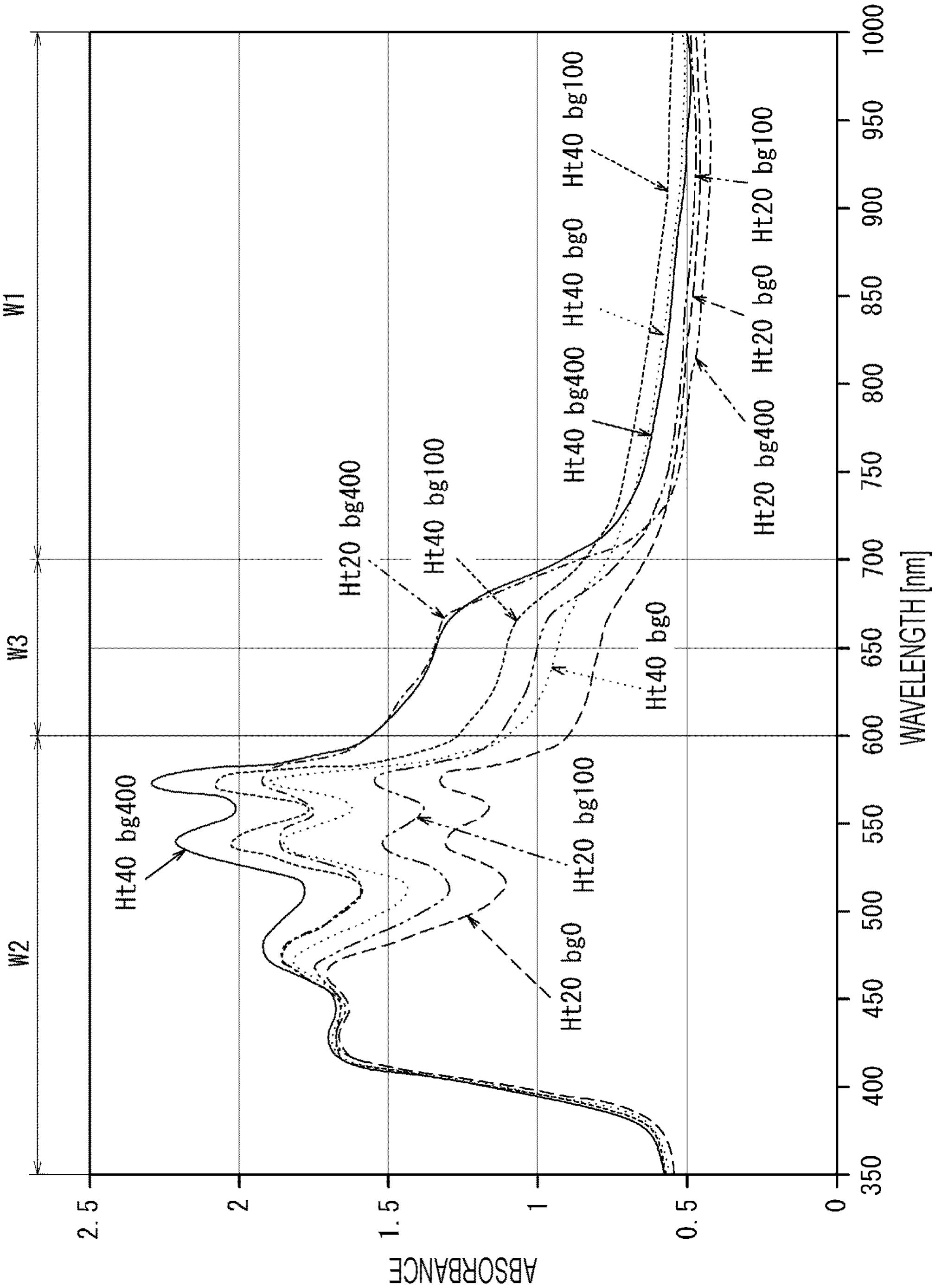
W2
W3
W1
ABSORBANCE
2.5
2
1.5
1
0.5
0
350
400
450
500
550
600
650
700
750
800
850
900
950
1000
WAVELENGTH [nm]
Ht40 bg400
Ht20 bg0
Ht20 bg100
Ht20 bg400
Ht40 bg100
Ht40 bg0
Ht40 bg400
Ht40 bg0
Ht40 bg100
Ht20 bg400
Ht20 bg0
Ht20 bg100

FIG. 11

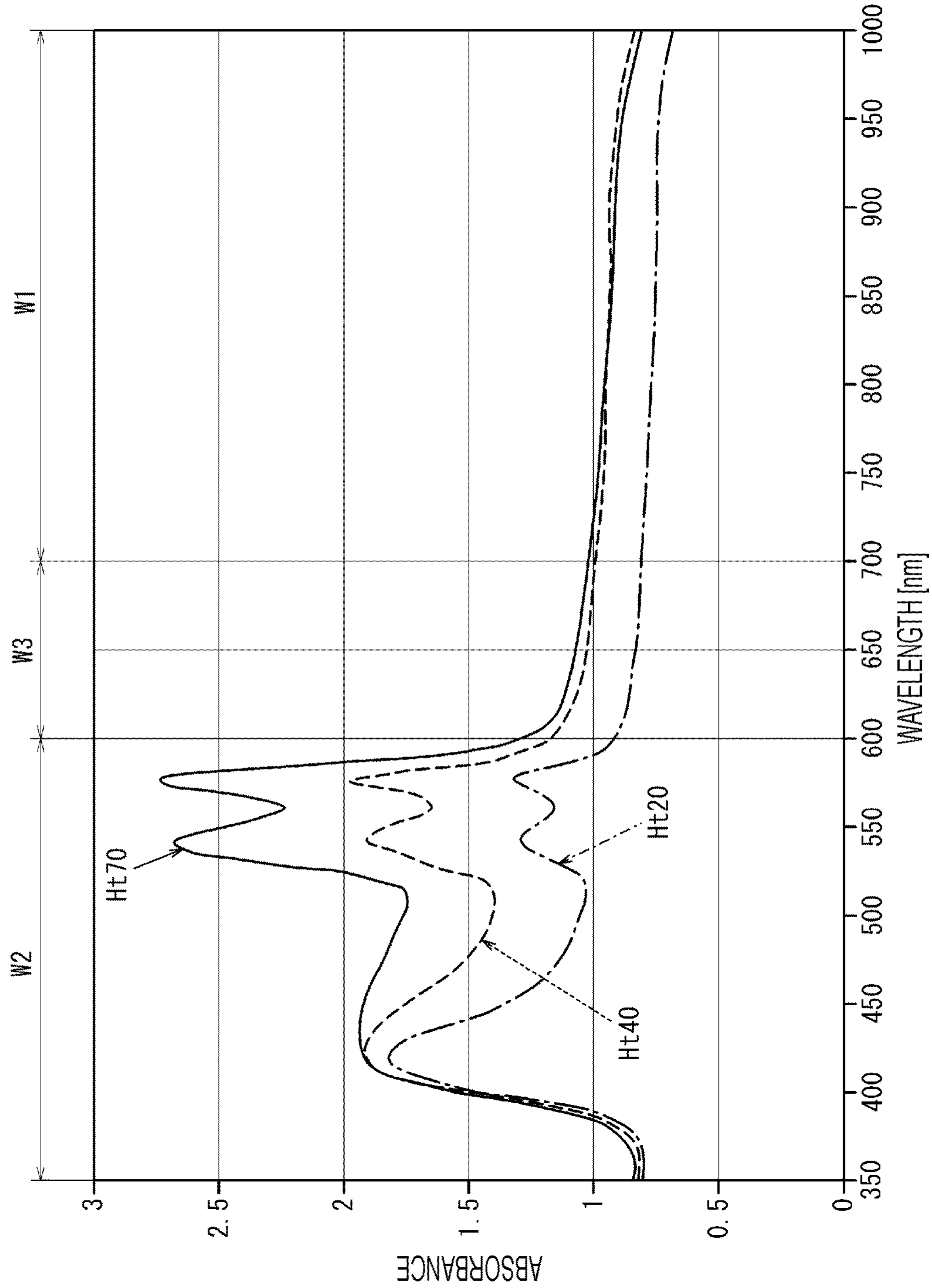
W2
W3
W1
Ht70
Ht40
Ht20
3
2.5
2
1.5
1
0.5
0
ABSORBANCE
350
400
450
500
550
600
650
700
750
800
850
900
950
1000
WAVELENGTH [nm]

FIG. 12

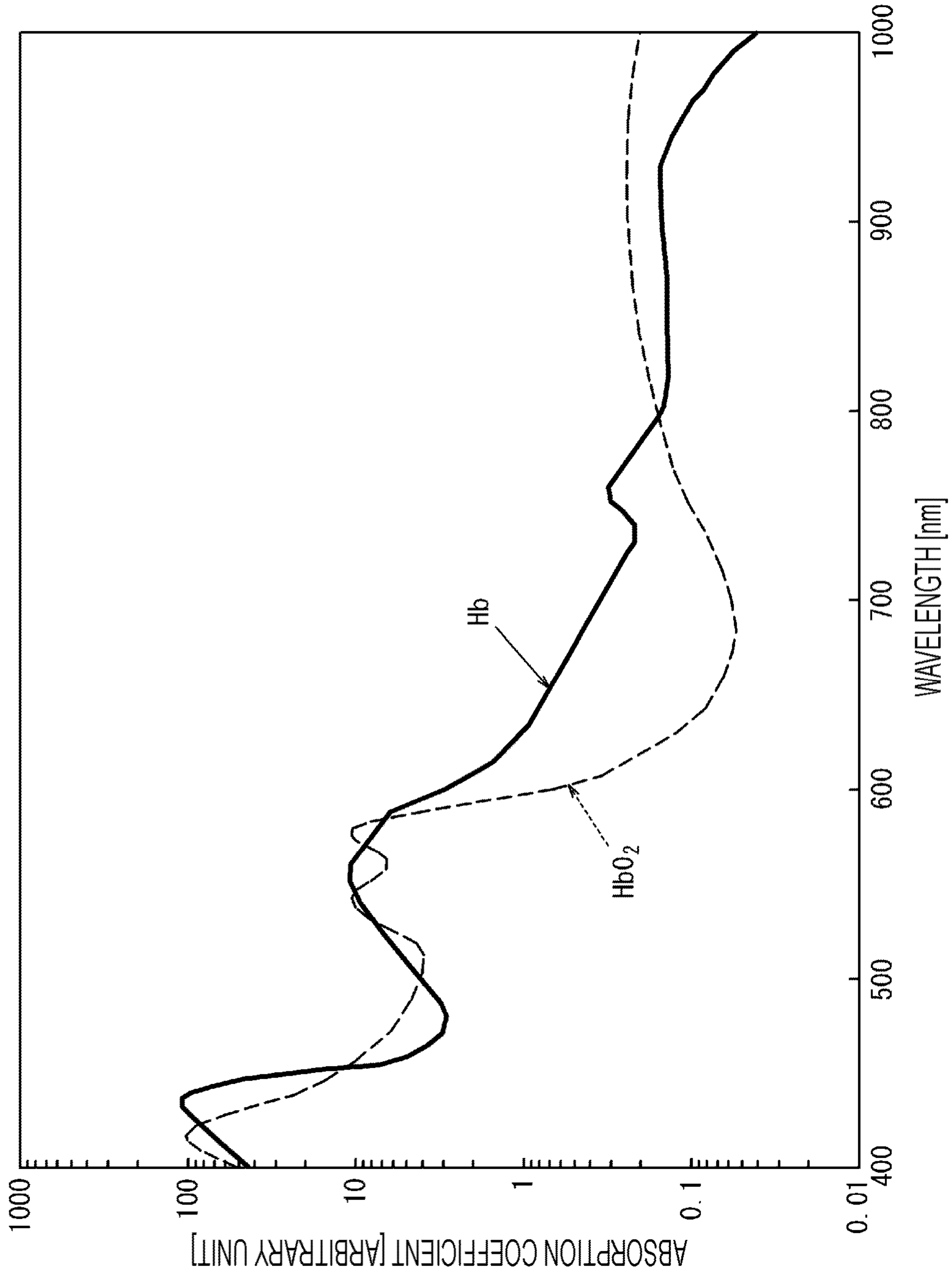
ABSORPTION COEFFICIENT [ARBITRARY UNIT]
1000
100
10
1
0. 1
0. 01
400
500
600
700
800
900
1000
WAVELENGTH [nm]
Hb
HbO2

# FIG. 13

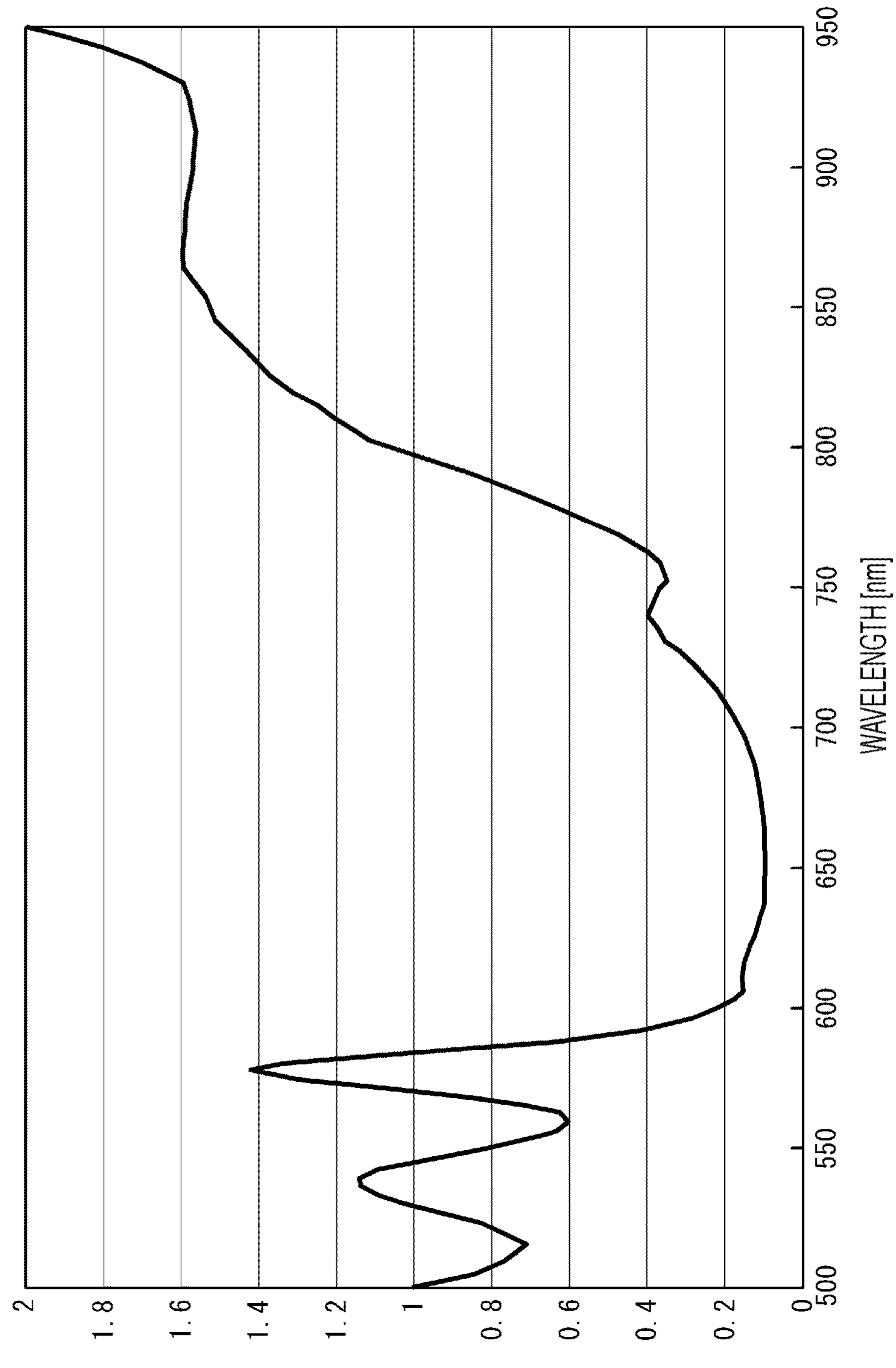

2
1.8
1.6
1.4
1.2
1
0.8
0.6
0.4
0.2
0
500
550
600
650
700
750
800
850
900
950
WAVELENGTH [nm]

FIG. 14

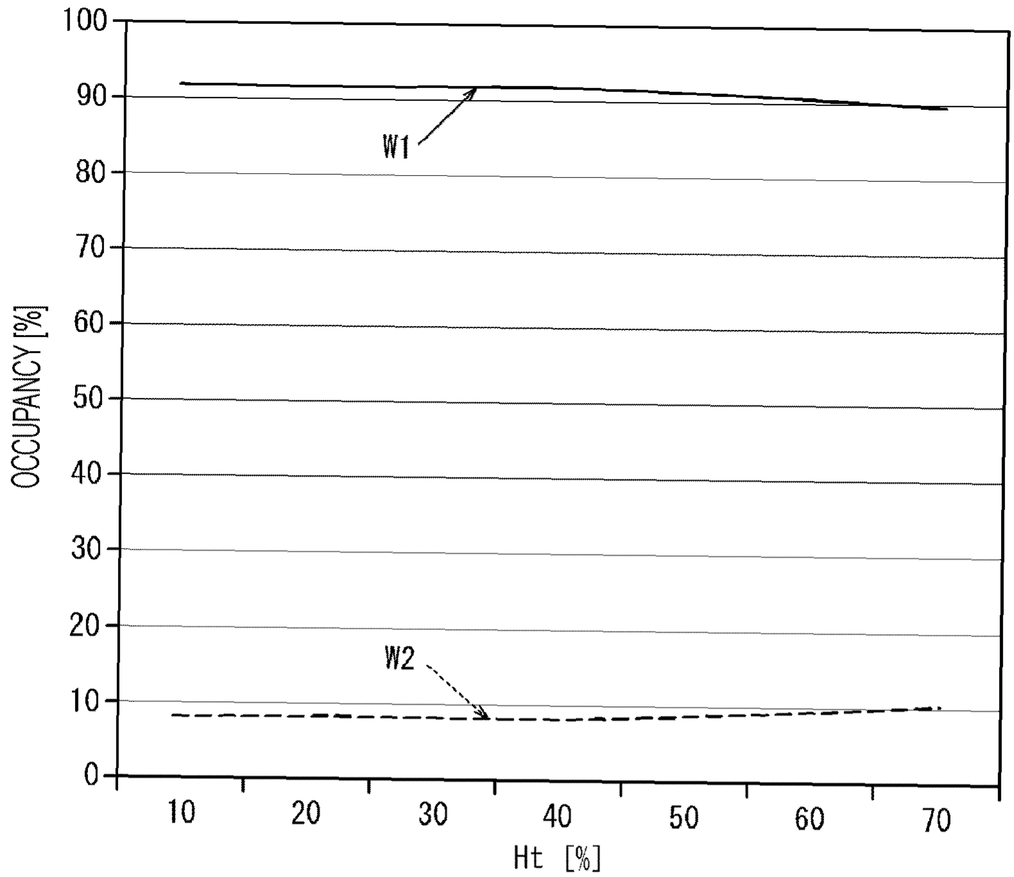
100
90
80
70
60
50
40
30
20
10
0
OCCUPANCY [%]
W1
W2
10
20
30
40
50
60
70
Ht [%]

FIG. 15

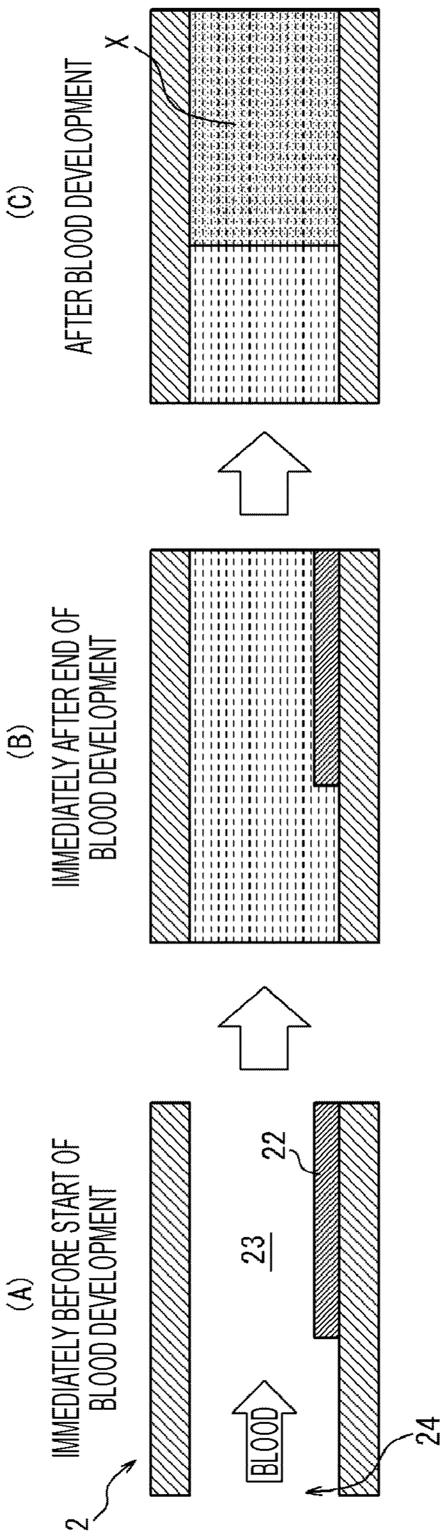
(A)
IMMEDIATELY BEFORE START OF BLOOD DEVELOPMENT
(B)
IMMEDIATELY AFTER END OF BLOOD DEVELOPMENT
(C)
AFTER BLOOD DEVELOPMENT
BLOOD
2
22
23
24
X

FIG. 16

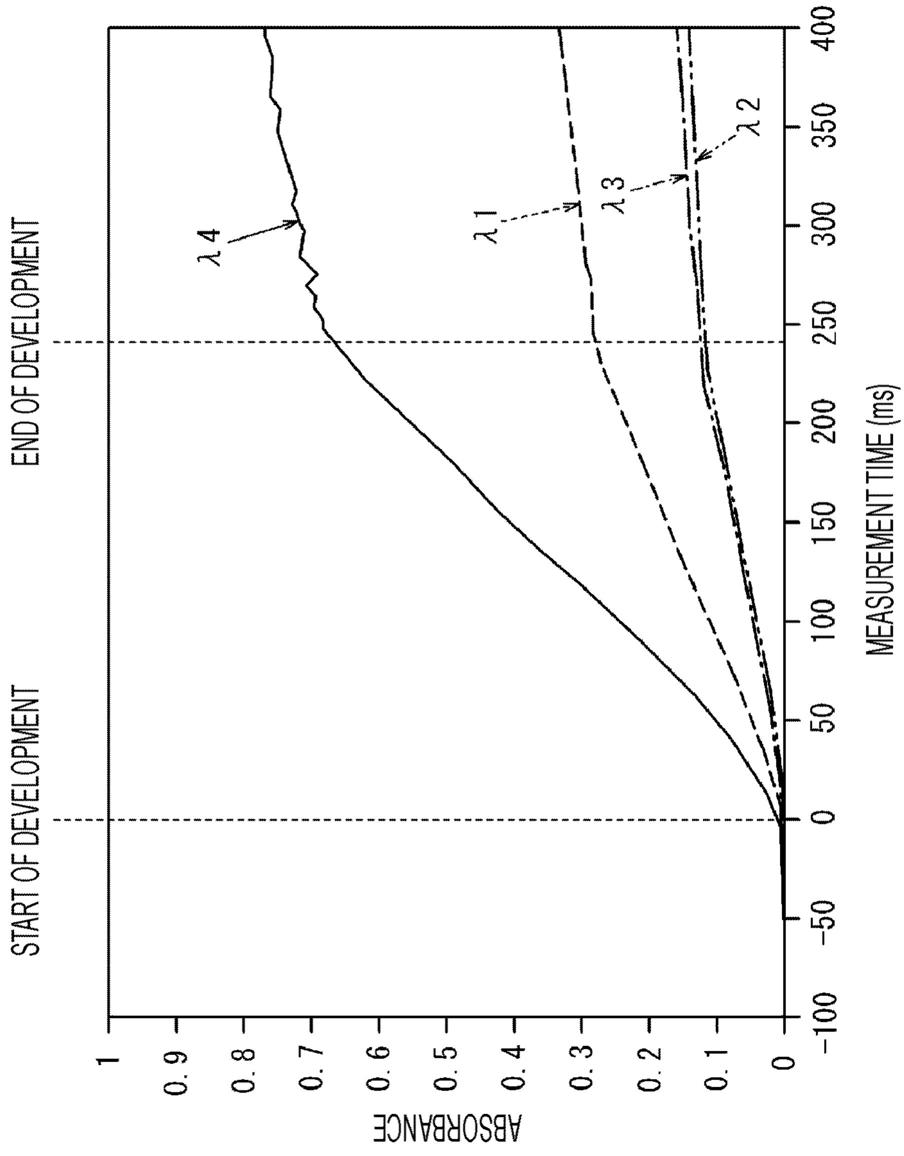
START OF DEVELOPMENT
END OF DEVELOPMENT
λ4
λ1
λ3
λ2
ABSORBANCE
0
0.1
0.2
0.3
0.4
0.5
0.6
0.7
0.8
0.9
1
-100
-50
0
50
100
150
200
250
300
350
400
MEASUREMENT TIME (ms)

FIG. 17

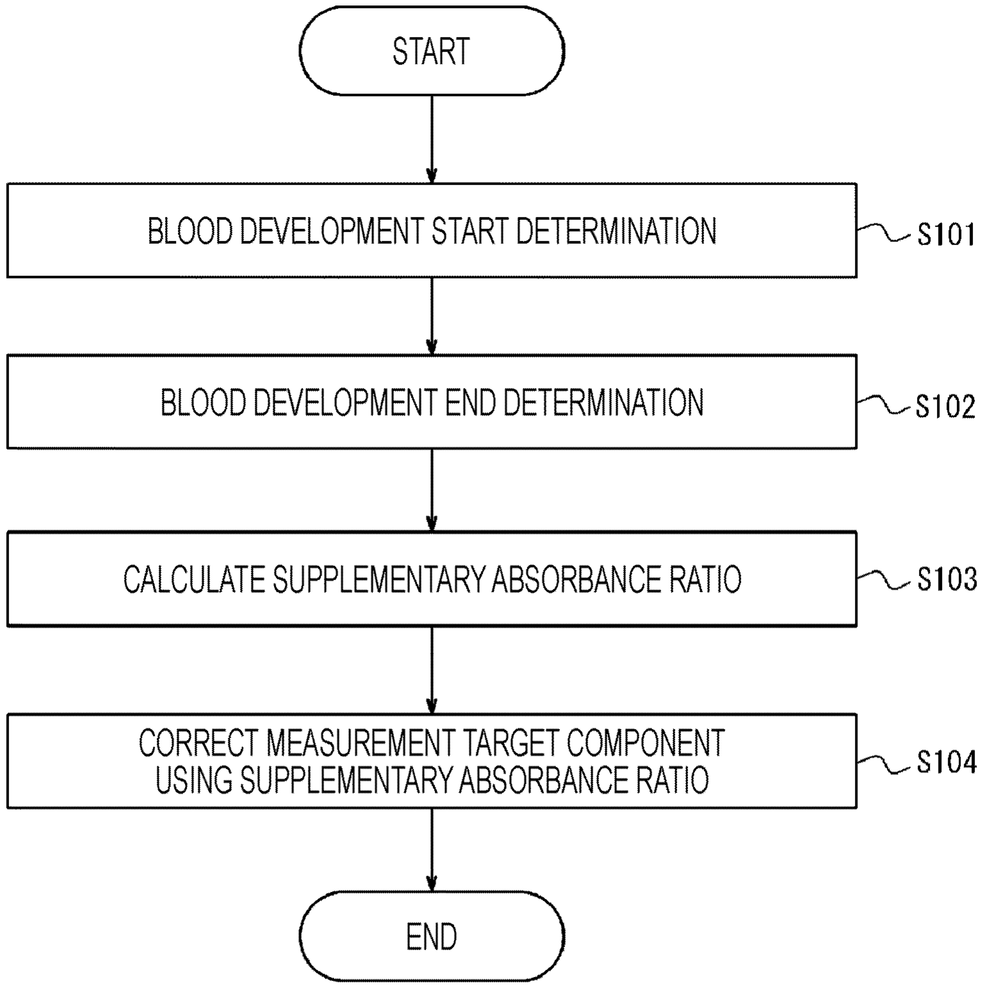
START
BLOOD DEVELOPMENT START DETERMINATION
S101
BLOOD DEVELOPMENT END DETERMINATION
S102
CALCULATE SUPPLEMENTARY ABSORBANCE RATIO
S103
CORRECT MEASUREMENT TARGET COMPONENT
USING SUPPLEMENTARY ABSORBANCE RATIO
S104
END

FIG. 18

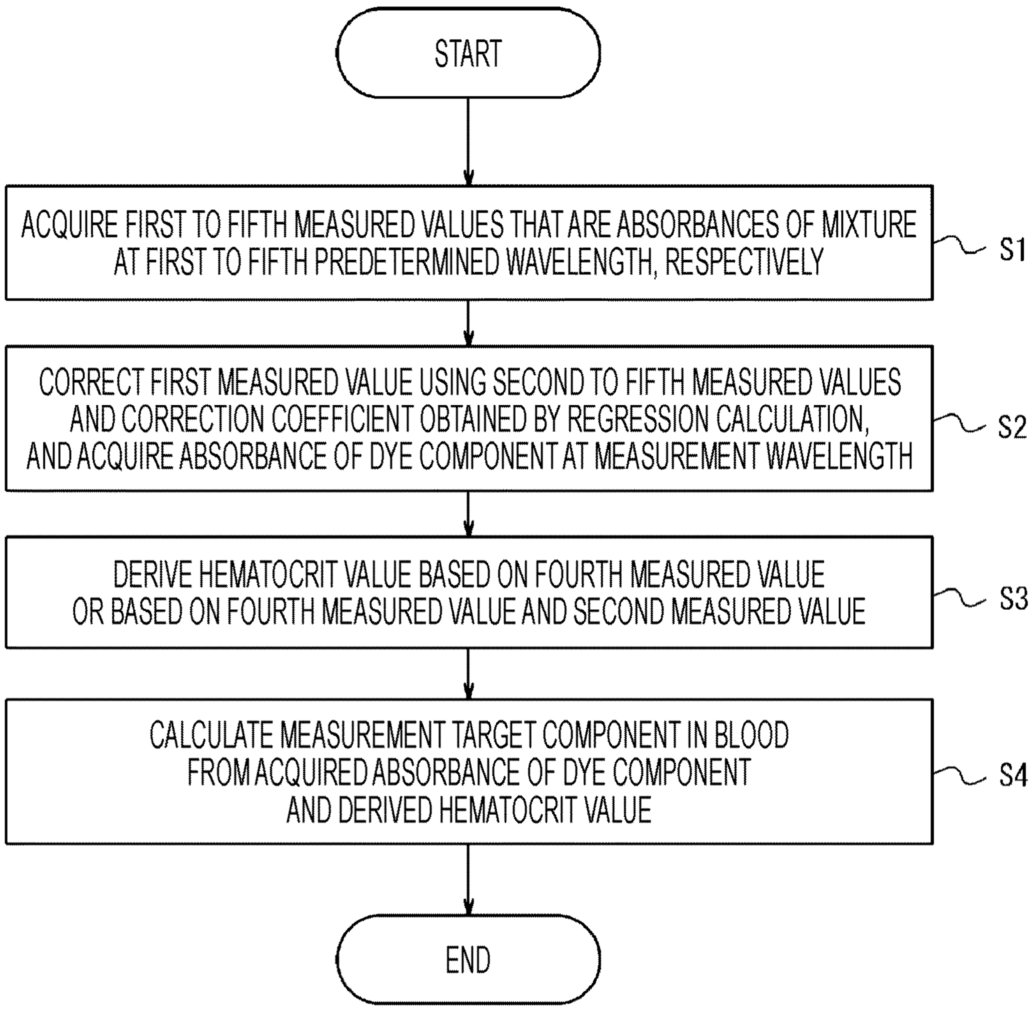
START
ACQUIRE FIRST TO FIFTH MEASURED VALUES THAT ARE ABSORBANCES OF MIXTURE AT FIRST TO FIFTH PREDETERMINED WAVELENGTH, RESPECTIVELY
S1
CORRECT FIRST MEASURED VALUE USING SECOND TO FIFTH MEASURED VALUES AND CORRECTION COEFFICIENT OBTAINED BY REGRESSION CALCULATION, AND ACQUIRE ABSORBANCE OF DYE COMPONENT AT MEASUREMENT WAVELENGTH
S2
DERIVE HEMATOCRIT VALUE BASED ON FOURTH MEASURED VALUE OR BASED ON FOURTH MEASURED VALUE AND SECOND MEASURED VALUE
S3
CALCULATE MEASUREMENT TARGET COMPONENT IN BLOOD FROM ACQUIRED ABSORBANCE OF DYE COMPONENT AND DERIVED HEMATOCRIT VALUE
S4
END

## INTERNATIONAL SEARCH REPORT

International application No.
**PCT/JP2024/000993**

**A. CLASSIFICATION OF SUBJECT MATTER**

***G01N 21/78***(2006.01)i; ***G01N 33/49***(2006.01)i; ***G01N 33/52***(2006.01)i
FI: G01N21/78 Z; G01N33/49 K; G01N33/52 A

According to International Patent Classification (IPC) or to both national classification and IPC

**B. FIELDS SEARCHED**

Minimum documentation searched (classification system followed by classification symbols)

G01N21/00-G01N21/83, G01N33/48-G01N33/98, G01N15/00-G01N15/1492, A61B5/00, A61K35/14, G01N7/00-G01N7/22, G01N31/00-G01N31/22

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII), JSTChina (JDreamIII), PubMed, Science Direct, ACS PUBLICATIONS, Nature, SCIENCE

**C. DOCUMENTS CONSIDERED TO BE RELEVANT**

| Category* | Citation of document, with indication, where appropriate, of the relevant passages | Relevant to claim No. |
|---|---|---|
| Y | WO 2017/122485 A1 (TERUMO KABUSHIKI KAISHA) 20 July 2017 (2017-07-20) paragraphs [0028]-[0081], fig. 1-5 | 1-6 |
| Y | WO 2019/138681 A1 (TERUMO KABUSHIKI KAISHA) 18 July 2019 (2019-07-18) paragraphs [0022]-[0091], fig. 1-9 | 1-6 |
| Y | US 2016/0157733 A1 (HUINNO, CO., LTD.) 09 January 2016 (2016-01-09) paragraphs [0008]-[0023], [0033]-[0064], fig. 1-8 | 1-6 |
| Y | WO 2018/173609 A1 (TERUMO KABUSHIKI KAISHA) 27 September 2018 (2018-09-27) paragraphs [0023]-[0146], fig. 1-19 | 2-5 |
| A | WO 2015/137074 A1 (TERUMO KABUSHIKI KAISHA) 17 September 2015 (2015-09-17) | 1-6 |
| A | JP 2009-233253 A (TERUMO KABUSHIKI KAISHA) 15 October 2009 (2009-10-15) | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 March 2024** | **26 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/000993**

**C. DOCUMENTS CONSIDERED TO BE RELEVANT**

| Category* | Citation of document, with indication, where appropriate, of the relevant passages | Relevant to claim No. |
|---|---|---|
| A | JP 05-095939 A (TERUMO KABUSHIKI KAISHA) 20 April 1993 (1993-04-20) | 1-6 |
| A | WO 2005/015181 A1 (GENPHARMTOX BIOTECH AG) 17 February 2005 (2005-02-17) | 1-6 |
| A | JP 56-098652 A (HERIGE GMBH) 08 August 1981 (1981-08-08) | 1-6 |
| A | JP 09-503866 A (MINNESOTA MINING AND MANUFACTURING COMPANY) 15 April 1997 (1997-04-15) | 1-6 |
| A | JP 07-191020 A (FUJI PHOTO FILM CO., LTD.) 28 July 1995 (1995-07-28) | 1-6 |
| A | JP 2002-543397 A (THE PROCTER & GAMBLE COMPANY) 17 December 2002 (2002-12-17) | 1-6 |
| A | JP 2005-501256 A (3M INNOVATIVE PROPERTIES COMPANY) 13 January 2005 (2005-01-13) | 1-6 |
| A | How to measure pO2 of tissue sealed in a transparent chamber? Development and construction of the first optrodes/optodes. SENSORS AND ACTUATORS B. 1998, vol. 51, pp. 5-11<br>fig. 1-8 | 1-6 |
| A | FLIM of luminescent oxygen sensors: clinical applications and results. SENSORS AND ACTUATORS B. 1998, vol. 51, pp. 171-175<br>fig. 1-4 | 1-6 |
| A | ラップトップ型 血液分析システム OPTI CCA. 05 March 2008, non-official translation (Laptop-Type Blood Analysis System OPTI CCA.)<br>Shape, Structure, Principles, etc. | 1-6 |

Form PCT/ISA/210 (second sheet) (July 2022)

**INTERNATIONAL SEARCH REPORT**
**Information on patent family members**

International application No.
**PCT/JP2024/000993**

| Patent document cited in search report | Publication date (day/month/year) | Patent family member(s) | Publication date (day/month/year) |
|---|---|---|---|
| WO 2017/122485 A1 | 20 July 2017 | CN 108474794 A | |
| WO 2019/138681 A1 | 18 July 2019 | US 2020/0340888 A1<br>paragraphs [0036]-[0105], fig. 1-9<br>EP 3722790 A1<br>CN 111630375 A | |
| US 2016/0157733 A1 | 09 January 2016 | WO 2015/167251 A1<br>EP 3138493 A1<br>AU 2015253957 A1<br>CN 106255461 A<br>KR 10-1512076 B1 | |
| WO 2018/173609 A1 | 27 September 2018 | US 2020/0011806 A1<br>paragraphs [0007]-[0019], [0040]-[0163], fig. 1-19<br>EP 3605069 A1<br>CN 110476055 A | |
| WO 2015/137074 A1 | 17 September 2015 | US 2016/0377634 A1<br>CN 106104259 A | |
| JP 2009-233253 A | 15 October 2009 | (Family: none) | |
| JP 05-095939 A | 20 April 1993 | (Family: none) | |
| WO 2005/015181 A1 | 17 February 2005 | DE 10335114 A1 | |
| JP 56-098652 A | 08 August 1981 | US 4336031 A<br>EP 31149 A1 | |
| JP 09-503866 A | 15 April 1997 | US 5518694 A<br>US 5462879 A<br>WO 1995/010766 A1<br>EP 760093 A1<br>CA 2171189 A1<br>CN 1133088 A | |
| JP 07-191020 A | 28 July 1995 | (Family: none) | |
| JP 2002-543397 A | 17 December 2002 | JP 2002-542843 A<br>JP 2002-542845 A<br>JP 2002-542846 A<br>JP 2003-517584 A<br>JP 2003-520615 A<br>JP 2002-519107 A<br>JP 2002-519109 A<br>JP 2010-207595 A<br>US 6713660 B1<br>US 6093869 A<br>US 2004/0172000 A1<br>US 6432097 B1<br>WO 2000/065084 A2<br>WO 2000/000233 A1<br>WO 2000/065083 A2<br>WO 2000/065096 A1<br>WO 2000/065347 A2<br>WO 2000/065348 A2<br>WO 2000/000145 A2 | |

Form PCT/ISA/210 (patent family annex) (July 2022)

**INTERNATIONAL SEARCH REPORT**
**Information on patent family members**

International application No.
**PCT/JP2024/000993**

| Patent document cited in search report | Publication date (day/month/year) | Patent family member(s) | Publication date (day/month/year) |
|---|---|---|---|
| | | WO 2000/000150 A1 | |
| | | EP 1091773 A1 | |
| | | EP 1173605 A1 | |
| | | EP 1173606 A1 | |
| | | EP 1173618 A1 | |
| | | EP 1173758 A1 | |
| | | EP 1173759 A1 | |
| | | EP 1091716 A1 | |
| | | EP 1091719 A1 | |
| | | AU 4842199 A1 | |
| | | AU 4492400 A1 | |
| | | AU 4665800 A1 | |
| | | AU 4665900 A1 | |
| | | AU 4802900 A1 | |
| | | AU 4803000 A1 | |
| | | CA 2367588 A1 | |
| | | CA 2370502 A1 | |
| | | KR 10-2002-0000877 A | |
| | | KR 10-2002-0007389 A | |
| | | AU 4850299 A1 | |
| | | AU 4962299 A1 | |
| | | CA 2333767 A1 | |
| | | CA 2336068 A1 | |
| | | CN 1313743 A | |
| | | KR 10-2001-0053233 A | |
| | | CA 2370509 A1 | |
| | | CA 2370739 A1 | |
| | | CA 2370936 A1 | |
| | | CN 1328476 A | |
| | | CN 1348501 A | |
| | | CN 1349562 A | |
| | | KR 10-2001-0053327 A | |
| JP 2005-501256 A | 13 January 2005 | US 2003/0099574 A1 | |
| | | WO 2003/019179 A1 | |
| | | EP 1423691 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018173609 A
- WO 2018051822 A